# EUROPEAN PATENT APPLICATION

(11) **EP 4 306 635 A1**
(43) Date of publication of application: **17.01.2024**
(21) Application number: 22307069.9
(22) Date of filing: 30.12.2022
(51) Int. Cl.: C12N 5/074

(54) **METHOD FOR OBTAINING INDUCED PLURIPOTENT STEM CELLS (IPSC)**

(30) Priority: 13.07.2022 EP 22306056
(71) Applicant: La Fondation Cardiométabolisme et Nutrition, 75013 Paris (FR)
(72) Inventor: NETCHINE, Irène, 94160 Saint Mande (FR); SOBRIER, Marie-Laure, 91330 Yerres (FR); SELENOU, Céline, 95330 Domont (FR); PHAM, Aurélie, 94120 Fontenay sous bois (FR); FONTAINE, Vincent, 91270 Vigneux sur seine (FR); MARTEAU, Sibylle, 92290 Chatenay-Malabry (FR)
(74) Representative: Novagraaf Technologies

(57) **Abstract**

The invention relates to a method for obtaining Induced pluripotent stem cells (iPSC) and to a kit for implementing the method for obtaining Induced pluripotent stem cells (iPSC).

The present invention finds applications in the biological research field, and diagnostic medical technical fields and also in veterinary technical fields.

## Description

### Technical field

The invention relates to a method for obtaining Induced pluripotent stem cells (iPSC).

The invention also relates to a kit for implementing the method for obtaining Induced pluripotent stem cells (iPSC).

The present invention finds applications in the biological research field, and diagnostic medical technical fields and also in veterinary technical fields.

The present invention can be used in particular in the pharmacological, medical and clinical fields.

In the description below, the references between square brackets ([ ]) refer back to the list of references presented at the end of the text.

### Prior art

Parental imprinting is an epigenetic mechanism that leads to the monoallelic expression of a subset of genes depending on their parental origin [1]. Differential methylation of Imprinting Control Regions (ICRs) is among the most studied mechanisms controlling such monoallelic expression. Imprinting disorders (IDs), caused by disturbances of imprinted genes, are a set of rare congenital diseases that mainly affect growth, metabolism and development [2]. Silver-Russell syndrome (SRS, MIM#180860), characterized by intra-uterine and postnatal growth retardation [3], is one such rare disease. Loss of methylation at the *H19*/*IGF2*:IG-DMR (differentially methylated region) (also called ICR1) at chromosome 11p15 is the principal molecular anomaly identified in these patients. Other imprinting regions are involved in human IDs, such as 15q11-13 for Prader-Willi syndrome (PWS, MIM#176266) and 14q32 for Temple syndrome (TS, MIM#616222), leading to diseases with overlapping features, regardless of the genomic region affected [4].

The low expression of imprinted genes in human tissues available for biological research, such as leucocytes and fibroblasts, is a major bottleneck for studying IDs and testing therapies at the cellular level [5]. In addition, tissues of interest in these diseases, such as cartilage, bone, liver, and brain, are not easily accessible. For all these reasons, many groups have attempted to develop cellular models to approach the mechanisms underlying the physiopathology of IDs [6-9].

However, the developed cellular models do not allow to clearly study the IDs and testing therapies at the cellular level. In particular, there is no accurate model to study the physiopathology of IDs or test therapeutic strategies.

There is therefore a real need in the prior art to find a means and/or a method which allows to clearly study the IDs and testing therapies.

Human induced pluripotent stem cells (iPSCs) are a cellular approach to model human diseases and complex genetic disorders due to their ability to self-renew and to differentiate into all three germ layers in culture [10,11]. Human iPSCs can be directly reprogrammed from somatic cells [12] and have been derived from patients with certain IDs [6,8,11,13,14]. However, epigenetic modifications, for example aberrant hypermethylation of imprinting control regions (ICRs) may appear during the reprogramming process and subsequent culture of iPSCs. In addition, the erasure of imprinting can appear during the reprogramming process and subsequent culture of iPSCs: loss of imprinting through hypermethylation of paternally methylated ICRs [7,9,10].

There is therefore a real need in the prior art to find a means and/or a method which allows to develop a cellular model without loss of imprinting, for example with balanced methylation in hiPSCs control at imprinted loci.

### Description of the invention

An objective of the present invention is specifically to meet these needs by providing a method for obtaining Induced pluripotent stem cells (iPSC) comprising the following steps:
**a.** culture of peripheral blood mononuclear cells (PBMCs) in a first culture medium M1
**b.** transduction or transformation of said PBMCs with a vector comprising at least one Yamanaka factor;
**c.** culture of the transduced or transformed cells obtained in step b) in hypoxic conditions,
**d.** seeding the cultured transduced or transformed cells obtained in step c) onto a matrix gel,
**e.** culture of seeded transduced or transformed cells of step d) in hypoxic conditions into a second culture medium M2,
**f.** replacing the culture medium M2 of the culture step e) by a third culture medium M3 comprising ascorbic acid,
**g.** Culturing the transduced or transformed cells obtained in step f) in hypoxic condition thus forming iPSC.

### Definitions

To facilitate an understanding of the present invention, a number of terms and phrases are defined below:
In the present, the term "comprise" can mean equally, on the one hand, "include", "contain" or "encompass" and, on the other hand, "constituted of" or "consist of".

As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof" "a mixture thereof" and such like following a listing, the use of "and/or" as part of a listing, a listing in a table, the use of "etc." as part of a listing, the phrase "such as," and/or a listing within brackets with "e.g.," or i.e., refers to any combination (e.g., any sub-set) of a set of listed components, and combinations and/or mixtures of related species and/or embodiments described herein though not directly placed in such a listing are also contemplated. Such related and/or like general(s), sub-general(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."

As used herein, the term "and/or" means any one of the items, any combination of the items, or all of the items with which this term is associated.

As used herein, the term "about" refers to a variation of ±5-10% of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., weight percents, proximate to the recited range that are equivalent in terms of the functionality of the individual ingredient, the composition, or the embodiment.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range (e.g., weight percents) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to," "at least," "greater than," "less than," "more than," "or more," and the like, include the number recited and such terms refer to ranges that can be subsequently broken down into subranges as discussed above. In the same manner, all ratios recited herein also include all sub-ratios falling within the broader ratio.

In the present "transfection," "transformation," or "transduction" refer to the introduction of one or more exogenous polynucleotides into a host cell, for example peripheral blood mononuclear cells (PBMCs), by using physical or chemical methods. It may be any transfection and/or transformation and/or transduction adapted method known to one skilled in the art. It may be for example, calcium phosphate DNA co-precipitation (see, e.g., Murray E. J. (ed.), Methods in Molecular Biology, Vol. 7, Gene Transfer and Expression Protocols, Humana Press (1991) [45]); DEAE-dextran; electroporation; cationic liposome-mediated transfection; tungsten particle-facilitated microparticle bombardment (Johnston, Nature, 346: 776-777 (1990) [46]); and strontium phosphate DNA co-precipitation (Brash et al., Mol. Cell Biol., 7: 2031-2034 (1987) [47]). In some embodiments, lipofection, nucleofection, or temporary membrane disruption (e.g., electroporation or deformation) can be used to introduce one or more exogenous polynucleotides into the host cell.

Advantageously, the method according to the invention makes it possible to obtain induced pluripotent stem cells (iPSC). In particular, the induced pluripotent stem cells (iPSC) obtained by the method of the invention are stable with a balanced methylation at imprinting loci.

In the present, by stable induced pluripotent stem cells (iPSC) means that the induced pluripotent stem cells (iPSC) have a phenotype and genotype which is not modified when reproduced, does not anarchically multiply, and can be induced for proliferation and differentiation in the three embryonic sheets. In other words, the obtained iPSCs from the process according to the invention have a genotype and a phenotype stable along the time and multiplication. Advantageously, the obtained iPSCs does not anarchically multiply, and can be induced for proliferation and differentiation in the three embryonic sheets.

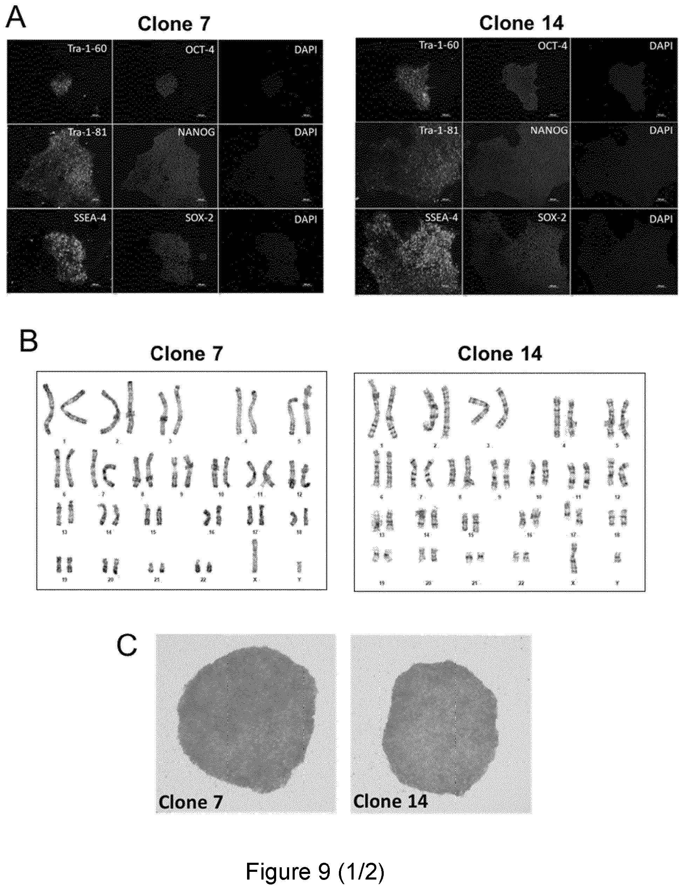

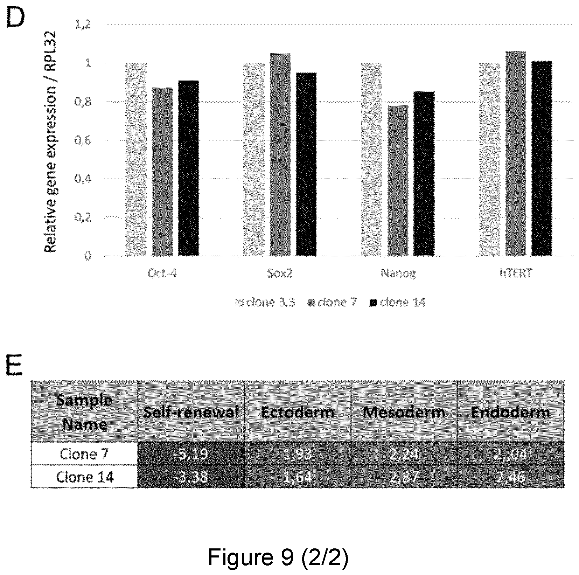

Advantageously, the inventors have demonstrated that the obtained iPSCs have a normal karyotype. Advantageously the inventors have verified and demonstrated that the karyotype and pluripotency markers attesting to the good quality of the obtained iPSC, for example as demonstrated in Figure 9.

In the present, by balanced methylation means that around 50%, for example from 45 to 55%, of methylation index (MI) is detected at each imprinted locus tested. The MI may be determined through Allele Specific Methylated Multiplex Real Time PCR (ASMM RT-QPCR) as developed by the Netchine laboratory and as disclosed in Azzi S, Steunou V, Rousseau A, Rossignol S, Thibaud N, Danton F, et al. Allele-specific methylated multiplex real-time quantitative PCR (ASMM RTQ-PCR), a powerful method for diagnosing loss of imprinting of the 11p15 region in Russell Silver and Beckwith Wiedemann syndromes. Hum Mutat. 2011 Feb;32(2):249-58. [42].

In the present invention, peripheral blood mononuclear cells that can be used in the method of the invention can be any peripheral blood mononuclear cells known to those skilled in the art. They may for example be peripheral blood mononuclear cells obtained from cell banks, for example originating from the Collection Nationale de Culture de Microorganismes [French National Collection of Microorganism Cultures] (CNCM) of the Institut Pasteur, 25 rue du Docteur Roux, F-75724 Paris Cedex 15. They may also be commercially available peripheral blood mononuclear cells. They may also be peripheral blood mononuclear cells isolated from a biological sample from an animal, preferably a mammal and/or from a human being, isolated beforehand. The peripheral blood mononuclear cells may be peripheral blood mononuclear cells isolated independently from a blood collection. The peripheral blood mononuclear cells may be isolated independently from blood collection from an individual, preferably a mammal and/or a human being. The peripheral blood mononuclear cells may be peripheral blood mononuclear cells isolated from a biological sample.

In the present document, "biological sample" is intended to mean any sample obtained from mammals, for example a mammal selected from the group comprising the orders Monotremata, Didelphimorphia, Paucituberculata, Microbiotheria, Notoryctemorphia, Dasyuromorphia, Peramelemorphia, Diprotodontia, Tubulidentata, Sirenia, Afrosoricida, Macroscelidea, Hyracoidea, Proboscidea, Cingulata, for example the armadillo, Pilosa, Scandentia, Dermoptera, Primates, Rodentia, Lagomorpha, Erinaceomorpha for example the hedgehog, Soricomorpha, Chiroptera, Pholidota, Carnivora, Perissodactyla, Artiodactyla and Cetacea. It may for example a biological sample from a human.

According to the invention, the biological sample may be a blood sample, plasma sample or any biological fluid. It may be for example a biological sample selected from the group comprising blood, plasma, urine, tears or tissue extracts. Preferably, the biological sample may be a blood sample, a plasma sample, a urine sample, preferably a plasma sample or a urine sample. It may also be a culture supernatant obtained from cultured cells in vitro.

According to the invention, the biological sample may originate from a mammal with imprinting defect diseases, for example the biological sample may originate from a mammal with Imprinting disorders (IDs), for example a biological sample originate from a mammal with Silver-Russell syndrome.

In the present invention, peripheral blood mononuclear cells that can be used in the method of the invention can be a peripheral blood mononuclear cells isolated from a biological sample originate from a mammal with a disease, for example selected from the group comprising Imprinting disorders (IDs), Silver-Russell syndrome, Temple syndrome, Beckwith-Wiedemann syndrome.

The process to isolate peripheral blood mononuclear cells from a biological sample may be any adapted process known from one skilled in the art. It may be for example the process disclosed in Gaignerie A, Lefort N, Rousselle M, Forest-Choquet V, Flippe L, Francois-Campion V, et al. Urine-derived cells provide a readily accessible cell type for feeder-free mRNA reprogramming. Sci Rep. 2018 25;8(1):14363[36].

In the present invention, the term "culture medium M1" or "medium M1" is intended to mean any medium known to those skilled in the art that are suitable for the culture of peripheral blood mononuclear cells. It may for example be a commercially available medium, for example a STEMPRO34^{®} basal medium plus supplements sold by the company Gibco. It may be for example an Iscove's modified Dulbecco's medium (IMDM) sold by the company Gibco further comprising in particular fetal bovine serum, stabilized glutamine, iron-saturated human transferrin, ferrous sulfate, ferric nitrate and insulin. It may be for example the culture medium disclosed in Majid Zamani et al. Humanized Culture Medium for Clinical-Grade Generation of Erythroid Cells from Umbilical Cord Blood CD34+ Cells Adv Pharm Bull, 2021, 11(2), 335-342, doi: 10.34172/apb.2021.031. It may be for example the culture medium of composition as defined in table 1.

**Table 1: example of composition of Iscove's modified Dulbecco's medium (IMDM)**

| **Components** | **mM** |
|---|---|
| **Amino Acids** | |
| Glycine | 0.4 |
| L-Alanine | 0.28089887 |
| L-Arginine hydrochloride | 0.39810428 |
| L-Asparagine (freebase) | 0.18939394 |
| L-Aspartic acid | 0.22556391 |
| L-Cystine 2HCl | 0.29201278 |
| L-Glutamic Acid | 0.5102041 |
| L-Glutamine | 4.0 |
| L-Histidine hydrochloride-H₂O | 0.2 |
| L-Isoleucine | 0.8015267 |
| L-Leucine | 0.8015267 |
| L-Lysine hydrochloride | 0.7978142 |
| L-Methionine | 0.20134228 |
| L-Phenylalanine | 0.4 |
| L-Proline | 0.3478261 |
| L-Serine | 0.4 |
| L-Threonine | 0.79831934 |
| L-Tryptophan | 0.078431375 |
| L-Tyrosine disodium salt | 0.46222222 |
| L-Valine | 0.8034188 |

| **Vitamins** | |
|---|---|
| Biotin | 5.327869E-5 |
| Choline chloride | 0.028571429 |
| D-Calcium pantothenate | 0.008385744 |
| Folic Acid | 0.009070295 |
| Niacinamide | 0.032786883 |
| Pyridoxal hydrochloride | 0.019607844 |
| Riboflavin | 0.0010638298 |
| Thiamine hydrochloride | 0.011869436 |
| Vitamin B12 | 9.594096E-6 |
| i-Inositol | 0.04 |

| **Inorganic Salts** | |
|---|---|
| CaCl₂ (anhyd.) | 1.4864864 |
| MgSO₄ (anhyd.) | 0.8139166 |
| KCl | 4.4 |
| KNO₃ | 7.524752E-4 |
| NaHCO₃ | 36.0 |
| NaCl | 77.67242 |
| NaH₂PO₄-H₂O | 0.9057971 |
| Na₂SeO₃-5H₂O | 9.8265904E-5 |

| **Other Components** | |
|---|---|
| D-Glucose (Dextrose) | 25.0 |
| HEPES | 25.033613 |
| Phenol Red | 0.039851222 |
| Sodium Pyruvate | 1.0 |
| thioglycerol or 2 mercaptoethanol | |

In the present invention, the medium M1 can also comprise at least one compound selected from the group comprising Stem Cell Factor (SCF), Fms-like tyrosine kinase 3 (FLT3), Interleukin 3 (IL3), Interleukin 6 (IL6) and erythropoietin (EPO).

In the present invention, the medium M1 can also comprise at least two compounds selected from the group comprising Stem Cell Factor (SCF), Fms-like tyrosine kinase 3 (FLT3), Interleukin 3 (IL3), Interleukin 6 (IL6) and erythropoietin (EPO).

In the present invention, the medium M1 can also comprise at least three compounds selected from the group comprising Stem Cell Factor (SCF), Fms-like tyrosine kinase 3 (FLT3), Interleukin 3 (IL3), Interleukin 6 (IL6) and erythropoietin (EPO).

In the present invention, the medium M1 can also comprise at least four compounds selected from the group comprising Stem Cell Factor (SCF), Fms-like tyrosine kinase 3 (FLT3), Interleukin 3 (IL3), Interleukin 6 (IL6) and erythropoietin (EPO).

In the present invention, the medium M1 can also comprise the compounds selected from the group comprising Stem Cell Factor (SCF), Fms-like tyrosine kinase 3 (FLT3), Interleukin 3 (IL3), Interleukin 6 (IL6) and erythropoietin (EPO).

In the present invention, the medium M1 can for example comprise a concentration of Stem Cell Factor (SCF) from 50 to 150 ng/mL, from 75 to 125 ng/mL, 100 ng/mL of medium M1.

In the present invention, the medium M1 can for example comprise a concentration of Fms-like tyrosine kinase 3 (FLT3), from 50 to 150 ng/mL, from 75 to 125 ng/mL, 100 ng/mL of medium M1.

In the present invention, the medium M1 can for example comprise a concentration of Interleukin 3 (IL3) from 5 to 40 ng/mL, from 10 to 30 ng/mL, 20 ng/mL of medium M1.

In the present invention, the medium M1 can for example comprise a concentration of Interleukin 6 (IL6) from 5 to 40 ng/mL, from 10 to 30 ng/mL, 20 ng/mL of medium M1.

In the present invention, the medium M1 can for example comprise a concentration of erythropoietin (EPO) from 1 to 5 U/mL, from 2 to 3 U/mL, 2.16 U/mL of medium M1.

Advantageously, the medium M1 comprises Stem Cell Factor (SCF), Fms-like tyrosine kinase 3 (FLT3), Interleukin 3 (IL3), Interleukin 6 (IL6) and erythropoietin (EPO).

For example, the culture medium M1 may be for example a medium as disclosed in table 2 below.

**Table 2: example of M1 medium**

| Iscove's modified Dulbecco's medium (IMDM) |
|---|
| 10% fetal bovine serum |
| 4 mM stabilized glutamine |
| 220 µg/mL iron-saturated human transferrin |
| 10 µg/mL ferrous sulfate |
| 100 ng/mL ferric nitrate |
| 20 µg/mL insulin |
| 100 ng/mL SCF |
| 100 ng/mL FLT3 |
| 20 ng/mL IL-3 |
| 20 ng/mL IL-6 |
| 2,16 U/mL EPO |

In the above table the concentrations are mentioned by weigh of the component to the volume of the culture medium or in percentage per volume of the culture medium.

In the present invention, the peripheral blood mononuclear cells (PBMCs) culture step a. can be carried out at a temperature comprised from 36 to 38°C, or equal to 37°C.

In the present invention, the peripheral blood mononuclear cells (PBMCs) culture time of step a. can be comprised from 5 to 21 days, from 5 to 15 days, or from 6 to 8 days.

In the present invention, the peripheral blood mononuclear cells (PBMCs) culture of step a. can be carried out under a controlled atmosphere comprising from 4% to 10% of CO₂, for example under an atmosphere comprising at least 5% of CO₂.

According to the invention, the peripheral blood mononuclear cells (PBMCs) culture step a. can be carried out in any suitable culture container known to those skilled in the art. It may be a petri dish, 12-well plate or a culture flask.

In the present the transformation of peripheral blood mononuclear cells (PBMCs) may be carried out by any adapted method known from one skilled in the art. Transformation of eukaryotic cells is a technique well known to those skilled in the art. It may be for example transduction, lipofection, electroporation, heat shock, or chemical methods (Vincent Fontaine, Laetitia-Duboscq-Bidot, Charlène Jouve, Matthieu Hamlin, Angélique Curjol, Véronique Briand, Philip Janiak, Jean-Sébastien Hulot, Marie-Pierre Pruniaux-Harnist, Philippe Charron, Eric Villard. Generation of iPSC line from MYH7 R403L mutation carrier with severe hypertrophic cardiomyopathy and isogenic CRISPR/Cas9 corrected control. Stem Cell Res, 2021, Apr, 52:102245.)

In the present the transduction of peripheral blood mononuclear cells (PBMCs) may be carried out by any adapted method known from one skilled in the art. Transduction of eukaryotic cells is a technique well known to those skilled in the art. (Vincent Fontaine, Laetitia-Duboscq-Bidot, Charlène Jouve, Matthieu Hamlin, Angélique Curjol, Véronique Briand, Philip Janiak, Jean-Sébastien Hulot, Marie-Pierre Pruniaux-Harnist, Philippe Charron, Eric Villard. Generation of iPSC line from MYH7 R403L mutation carrier with severe hypertrophic cardiomyopathy and isogenic CRISPR/Cas9 corrected control. Stem Cell Res, 2021, Apr, 52:102245.)

According to the invention, the vector may be any vector known from one skilled in the art adapted for the transformation of eucaryotic or prokaryotic cells. It may be for example a commercially available vector. It may be for example, a plasmid vector, for example pCXLE-hOCT3/4-shp53-F (Oct3/4) for example as shown in Figure 10, pCXLE-hSK (Klf-4, Sox2) for example as shown in Figure 11, pCXLE-hUL (L-myc and Lin28) as shown in Figure 12. It may be for example, a virus vector, for example Sendai virus, for example Sendai virus commercialized by ThermoFischer. Preferably, the vector is a virus vector, for example Sendai virus.

According to the invention, the vector may comprise at least one Yamanaka factors. It may be for example a factor selected from the group comprising OCT4, SOX2, KLF4 and c-Myc.

According to the invention, the vector may comprise at least one Yamanaka factors selected from the group comprising OCT4, SOX2, KLF4 and c-Myc.

According to the invention, the vector may comprise at least two Yamanaka factors selected from the group comprising OCT4, SOX2, KLF4 and c-Myc.

According to the invention, the vector may comprise at least three Yamanaka factors selected from the group comprising OCT4, SOX2, KLF4 and c-Myc.

According to the invention, the vector may comprise Yamanaka factors selected from the group comprising OCT4, SOX2, KLF4 and c-Myc.

According to the invention, the vector may express in the transformed or transfected cells at least one Yamanaka factor. For example, the vector may express in the transformed or transfected cells at least one Yamanaka factor selected from the group comprising OCT4, SOX2, KLF4 and c-Myc.

For example, the vector may express in the transformed or transfected cells at least two Yamanaka factor selected from the group comprising OCT4, SOX2, KLF4 and c-Myc.

For example, the vector may express in the transformed or transfected cells at least three Yamanaka factor selected from the group comprising OCT4, SOX2, KLF4 and c-Myc.

Advantageously, the vector may comprise at least the factor selected from the group comprising OCT4, SOX2, KLF4 and c-Myc.

Advantageously, the vector may express in the transformed or transfected cells OCT4, SOX2, KLF4 and c-Myc.

In the present OCT4 means the factor coded by the gene with Gene ID: 5460 and ENSG00000204531.

In the present SOX2 means the factor coded by the gene with Gene ID: 6657 and ENSG00000181449.

In the present KLF4 means the factor coded by the gene with Gene ID: 9314 and ENSG00000136826.

In the present c-Myc means the factor coded by the gene with Gene ID: 4609 and ENSG00000136997.

In the present invention, the transduction or transformation of peripheral blood mononuclear cells (PBMCs) step b. can be carried out at a temperature comprised, from 36 to 38°C, or equal to 37°C.

In the present invention, the transduction or transformation of peripheral blood mononuclear cells (PBMCs) step b can be carried out under a controlled atmosphere comprising from 4% to 10% of CO₂, for example under an atmosphere comprising at least 5% of CO₂.

According to the invention, the transduction or transformation of peripheral blood mononuclear cells (PBMCs) step b can be carried out in any suitable culture container known to those skilled in the art. It may be a petri dish, 12 well plate, 24-well plate or a culture flask.

According to the invention, the transduction or transformation of peripheral blood mononuclear cells (PBMCs) step b can be carried out in culture medium M1. Advantageously, the transduction of peripheral blood mononuclear cells (PBMCs) step b can be carried out in culture medium M1 comprising Stem Cell Factor (SCF), Fms-like tyrosine kinase 3 (FLT3), Interleukin 3 (IL3), Interleukin 6 (IL6) and erythropoietin (EPO). The medium M1 comprising Stem Cell Factor (SCF), Fms-like tyrosine kinase 3 (FLT3), Interleukin 3 (IL3), Interleukin 6 (IL6) and erythropoietin (EPO) may be as disclosed above.

According to the invention, the method may comprise before step c) a seeding step b' of the transduced or transformed cells.

According to the invention, the seeding may be carried out onto any surface adapted surface. It may be for example onto a surface of a culture container. The container may be any culture container known to those skilled in the art. It may be a petri dish, 12-well plate, 24-well plate or a culture flask.

In the present invention, the seeding of step b'. can be carried out by any method known to those skilled in the art. It may for example be an application, for example by sprinkling a culture medium comprising the transduced or transformed cells onto a surface, by deposition by subculturing the cells on a surface, by pouring of a culture medium comprising the cells in suspension, or by 3D culture.

In the present invention, the seeding of step b', for example of a surface of a culture container, can be carried out with a concentration of cells from 400000 to 500000 cells/ml, for example 450000 cells/ml.

In the present invention, the transduced or transformed cells culture step c. can be carried out at a temperature comprised from 36 to 38°C, or equal to 37°C.

In the present invention, the transduced or transformed cells culture time of step c. can be comprised from 3 to 30 days, from 7 to 20 days

In the present invention, the transduced or transformed cells culture step c. can be carried out under a controlled atmosphere comprising from 5% to 10% of CO₂, for example under an atmosphere comprising at least 5% of CO₂.

In the present invention, the transduced or transformed cells culture step c. can be carried out under hypoxic condition wherein the oxygen (O₂) level is from 3% to 17%, preferably from 3 to 7%, more preferably at 5%.

According to the invention, the transduced or transformed cells culture step c. can be carried out in culture medium M1. Advantageously, the transduced or transformed cells culture step c. can be carried out in medium M1 comprising Stem Cell Factor (SCF), Fms-like tyrosine kinase 3 (FLT3), Interleukin 3 (IL3), Interleukin 6 (IL6) and erythropoietin (EPO). The medium M1 comprising Stem Cell Factor (SCF), Fms-like tyrosine kinase 3 (FLT3), Interleukin 3 (IL3), Interleukin 6 (IL6) and erythropoietin (EPO) may be as disclosed above.

In the present invention, the medium M1 and/or each of the components thereof may be of clinical grade.

According to the invention, the transduced or transformed cells culture step c. can be carried out in any suitable culture container known to those skilled in the art. It may be a petri dish, 12-well plate or a culture flask.

In the present invention, step c. of the transduced or transformed cells obtained in step b) in hypoxic conditions can comprise
- c1 culturing the transduced cells obtained in step b) for 18 to 28 hours, preferably 24 hours, in hypoxic conditions,
- c2 elimination of the vector from the culture medium,
- c3 resuspending the transduced cells into culture medium M2,
- c4 culturing the resuspended cells of step c3 for 24 to 96 hours, preferably 72 hours.

In the present invention, the culture step c1. can be carried out under hypoxic condition wherein the oxygen (O₂) level is from 3% to 17%, preferably from 3 to 7%, more preferably at 5%.

In the present invention, the elimination of the vector from the culture medium step c2 can be carried out by any method known from one skilled in the art. It may be for example by pelleting the cells and removing the culture medium. The pelleting can be carried out by any method known to those skilled in the art. It can be for example be a sedimentation or a centrifugation at a speed of 1000 to 1300 revolutions per minute, for example equal to 1160 revolutions per minute. According to the invention, the pelleting can be carried out by any device known to those skilled in the art. It may for example be a rotary centrifuge sold by the company Eppendorf or Thermofisher. The removing step mays be carried out by any suitable method known to on skilled in the art. It may be for example carried out by suctioning of the medium, or turning the container upside-down in order to remove the culture medium.

In the present invention, the culture step c3. can be carried out in hypoxic conditions wherein the oxygen (O₂) level is from 3% to 17%, preferably from 3 to 7%, more preferably at 5%.

In the present invention, the term "matrix gel" is intended to mean any matrix that is known to those skilled in the art and that can be seeded with cells. It may be for example a matrix comprising at least compound selected from the group comprising collagen type I, collagen type IV, Entactin and laminin. It may be for example a commercially available matrix, for example the matrix commercialized under the reference Matrigel^{®} by Corning, the matrix commercialized under the reference BME2^{®} by Biotechne, the matrix commercialized under the reference Geltrex^{®} by ThermoFisher. The matrix may also further comprise Heparan sulfate proteoglycans and/or Perlecan. It may be for example a matrix comprising collagen type I, collagen type IV, Perlecan, Entactin and laminin.

In the present invention, the seeding of step d. can be carried out by any method known to those skilled in the art. It may for example be an application, for example by sprinkling a culture medium comprising the transduced or transformed cells onto the matrix, for example by deposition by subculturing the cells on the matrix, by pouring of a culture medium comprising the cells in suspension, or by 3D culture.

In the present invention, the seeding of step d. of a gel matrix can be carried out with a concentration of cells from 450000 to 500000 cells/ml, for example 475000 cells/ml.

In the present invention, the method of the invention may comprise, before the seeding step d., a step d1 of centrifugation and a step d2 of resuspension of centrifuged cells in the medium M2.

The centrifugation step d1 can at a speed of 1000 to 1300 revolutions per minute, for example equal to 1160 revolutions per minute.

In the present invention, the term "culture medium M2" or "medium M2" is intended to mean any medium known to those skilled in the art that adapted for the culture of transduced or transformed cells and/or seeded onto a matrix of transduced or transformed cells. It may for example be a commercially available medium or a mix of commercially available medium, for example a medium selected for the group comprising STEMPRO34^{®} SFM basal medium sold by the company Gibco, STEMPRO34^{®} supplement sold by the company Gibco. It may be for example culture medium as disclosed in table 3 or table 4 below:

**Table 3: example of medium M2**

| **Composants** | **Volumes** |
|---|---|
| Stem-Pro-34^{®} SFM basal Medium | 50 mL |
| Stem-Pro-34^{®} Supplement | 1.25mL |

It may be for example the culture medium of composition as defined in table 1 comprising at least one compound selected from the group comprising fetal bovine serum, glutamine, iron-saturated human transferrin, ferrous sulfate, ferric nitrate and insulin.

The medium M2 can also comprise at least two compounds selected from the group comprising fetal bovine serum, glutamine, iron-saturated human transferrin, ferrous sulfate, ferric nitrate and insulin.

The medium M2 can also comprise at least three compounds selected from the group comprising fetal bovine serum, glutamine, iron-saturated human transferrin, ferrous sulfate, ferric nitrate and insulin.

The medium M2 can also comprise at least four compounds selected from the group comprising fetal bovine serum, glutamine, iron-saturated human transferrin, ferrous sulfate, ferric nitrate and insulin.

The medium M2 can also comprise at least five compounds selected from the group comprising fetal bovine serum, glutamine, iron-saturated human transferrin, ferrous sulfate, ferric nitrate and insulin.

The medium M2 can also comprise at least six compounds selected from the group comprising fetal bovine serum, glutamine, iron-saturated human transferrin, ferrous sulfate, ferric nitrate and insulin.

The medium M2 can also comprise the compounds selected from the group comprising fetal bovine serum, glutamine, iron-saturated human transferrin, ferrous sulfate, ferric nitrate and insulin.

The medium M2 can for example comprise a concentration of fetal bovine serum from 5% to 10%, for example 10% by weight to the volume of the culture medium

The medium M2 can for example comprise a concentration of glutamine from 2mM to 6mM, for example 4 mM.

The medium M2 can for example comprise a concentration of iron-saturated human transferrin from 150µg/mL to 290µg/mL, for example 220 µg/mL of the culture medium.

The medium M2 can for example comprise a concentration of ferrous sulfate from 5µg/mL to 15µg/mL, for example 10 µg/mL of the culture medium.

The medium M2 can for example comprise a concentration of ferric nitrate from 50ng/mL to 150ng/mL, for example 100 ng/mL of the culture medium.

The medium M2 can for example comprise a concentration of insulin from 10µg/mL to 30µg/mL, for example 20 µg/mL of the culture medium.

Medium M2 may be for example culture medium as disclosed in table 4.

**Table 4: example of medium M2**

| Iscove's modified Dulbecco's medium (IMDM) |
|---|
| 10% fetal bovine serum |
| 4 mM stabilized glutamine |
| 220 µg/mL iron-saturated human transferrin |
| 10 µg/mL ferrous sulfate |
| 100 ng/mL ferric nitrate |
| 20 µg/mL insulin |

In table 4 the concentrations are mentioned by weigh of the component to the volume of the culture medium or in percentage per volume of the culture medium.

In the present invention, the medium M2 and/or each of the components thereof may be of clinical grade.

In the present invention, the seeded transduced or transformed cells culture step e) can be carried out at a temperature comprised from 36 to 38°C, or equal to 37°C.

In the present invention, the seeded transduced or transformed cells culture time of step e) can be comprised from 1 to 10 days, from 2 to 7 days, equal to 3 days.

In the present invention, the seeded transduced or transformed cells culture step e) can be carried out under a controlled atmosphere comprising from 5% to 10% of CO₂, for example under an atmosphere comprising at least 5% of CO₂.

In the present invention, the seeded transduced or transformed cells culture step e) can be carried out in hypoxic condition wherein the oxygen (O₂) level is from 3% to 17%, preferably from 3 to 7%, more preferably at 5%.

In the present invention, in step f, the replacement of the culture medium M2 by culture medium M3 may be carried out by any suitable method known from one skilled in the art. It may comprise for example a removing step of the medium M2 and an adding step of medium M3. The removing step may be carried out by any suitable method known to on skilled in the art. It may be for example carried out by suctioning of the medium, or turning the container upside-down in order to remove the culture medium.

In the present invention, the term "culture medium M3" or "Medium M3" is intended to mean any culture medium known from one skilled in the art adapted for the culture of transduced or transformed cells. It may be for example a culture medium comprising amino acids, vitamins, inorganic salts, dextrose, hypoxanthine, linoleic acid, lipoic acid, phenol red, putrescine, sodium, pyruvate and thymidine. It may be for example a culture medium comprising at least one amino acid selected from the group comprising glycine, l-alanine, l-arginine hydrochloride, l-asparagine, l-aspartic acid, l-cysteine, l-cystine, l-glutamic acid, l-glutamine, l-histidine, l-isoleucine, l-leucine, l-lysine, l-methionine, l-phenylalanine, l-proline, l-serine, l-threonine, l-tryptophan, l-tyrosine, l-valine. It may be for example a culture medium comprising at least one vitamin selected from the group comprising biotin, choline chloride, D-Calcium pantothenate, folic acid, niacinamide, pyridoxine, riboflavin, thiamine hydrochloride, vitamin B12 and i-Inositol. It may be for example a culture medium comprising at least one inorganic salt selected from the group comprising CaCl₂, CuSO₄, Fe(NO₃)₃, FeSO₄, Magnesium Chloride, MgSO₄, KCl, NaHCOs, NaCl, Na₂HPO₄, NaH₂PO₄, ZnSO₄. Medium M3 can also comprise at least one compound selected from the group comprising bovine serum albumin fraction V, γ-aminobutyric acid, thiamine hydrochloride, glutathione, insulin, bovine holo-transferrin, 2-mercaptoethanol, L-ascorbic acid 2-phosphate magnesium, sodium selenite, pipecolic acid, L-glutamine, lithium chloride, MEM NEAA, Transforming Growth Factor beta 1 (TGFβ1) and Fibroblast Growth Factor 2 (FGF-2).

It may be for example be a commercially available medium, for example mTeSR1@ sold by the company Gibco. It may for example be a commercially available medium, for example a Dulbecco's modified Eagle's minimal essential medium plus Nutrient Mixture Ham F12 (DMEM/F12) sold by the company Gibco comprising in particular a mixture of bovine serum albumin fraction V, γ-aminobutyric acid, Thiamine hydrochloride, reduced Glutathione, insulin, bovine holo-transferrin, 2-mercaptoethanol, l-ascorbic acid 2-phosphate magnesium, sodium selenite, pipecolic acid, l-glutamine, lithium chloride, MEM NEAA, NaHCOs, TGFβ1,FGF-2. It may be for example the culture medium disclosed in Ludwig, T., Bergendahl, V., Levenstein, M. et al. Feeder-independent culture of human embryonic stem cells. Nat Methods 3, 637-646 (2006). https://doi.org/10.1038/nmeth902 [44].

**Table 5: example of composition of DMEM/F12 medium**

| **Components** | **mM** |
|---|---|
| **Amino Acids** | |
| Glycine | 0.25 |
| L-Alanine | 0.049999997 |
| L-Arginine hydrochloride | 0.69905216 |
| L-Asparagine-H2O | 0.05 |
| L-Aspartic acid | 0.05 |
| L-Cysteine hydrochloride-H2O | 0.09977272 |
| L-Cystine 2HCl | 0.09996805 |
| L-Glutamic Acid | 0.05 |
| L-Glutamine | 2.5 |
| L-Histidine hydrochloride-H2O | 0.14990476 |
| L-Isoleucine | 0.41580153 |
| L-Leucine | 0.45076334 |
| L-Lysine hydrochloride | 0.4986339 |
| L-Methionine | 0.11570469 |
| L-Phenylalanine | 0.2150303 |
| L-Proline | 0.15 |
| L-Serine | 0.25 |
| L-Threonine | 0.44915968 |
| L-Tryptophan | 0.04421569 |
| L-Tyrosine disodium salt dihydrate | 0.21375479 |
| L-Valine | 0.4517094 |

| **Vitamins** | |
|---|---|
| Biotin | 1.4344263E-5 |
| Choline chloride | 0.06414285 |
| D-Calcium pantothenate | 0.0046960167 |
| Folic Acid | 0.0060090707 |
| Niacinamide | 0.016557377 |
| Pyridoxine hydrochloride | 0.009771844 |
| Riboflavin | 5.824468E-4 |
| Thiamine hydrochloride | 0.0064391694 |
| Vitamin B12 | 5.0184503E-4 |
| i-Inositol | 0.07 |

| **Inorganic Salts** | |
|---|---|
| CaCl2 (anhyd.) | 1.0504504 |
| CuSO4-5H2O | 5.2E-6 |
| Fe(NO3)₃"9H2O | 1.2376238E-4 |
| FeSO4-7H2O | 0.0015 |
| Magnesium Chloride (anhydrous) | 0.30147368 |
| MgSO4 (anhyd.) | 0.407 |
| KCI | 4.1573334 |
| NaHCO3 | 29.02381 |
| NaCl | 120.61207 |
| Na2HPO4 anhydrous | 0.50014085 |
| NaH2PO4-H2O | 0.45289856 |
| ZnSO4-7H2O | 0.0015 |

| **Other Components** | |
|---|---|
| D-Glucose (Dextrose) | 17.505556 |
| Hypoxanthine Na | 0.015031448 |
| Linoleic Acid | 1.4999999E-4 |
| Lipoic Acid | 5.097087E-4 |
| Phenol Red | 0.021519661 |
| Putrescine 2HCl | 5.031056E-4 |
| Sodium Pyruvate | 0.5 |
| Thymidine | 0.0015082645 |

The culture medium M3 may be for example a medium as disclosed in table 6 below.

**Table 6: example of composition of medium M3**

| **Ingredients** |
|---|
| DMEM/F12 |
| Bovine serum albumin fraction V |
| γ-aminobutyric acid |
| Thiamine hydrochloride |
| Reduced Glutathione |
| Insulin |
| Trace elements B |
| Trace elements C |
| Bovine holo-Transferrin |
| 2-mercaptoethanol |
| L-ascorbic acid 2-phosphate magnesium |
| Chemically defined lipid concentrate |
| Sodium selenite |
| Pipecolic acid |
| L-glutamine |
| Lithium chloride |
| MEM NEAA |
| NaHCO₃ |
| TGFβ1 |
| FGF-2 |

In the present invention, the culture medium M3 can also comprise ascorbic acid.

In the present invention, the culture medium M3 can for example comprise a concentration ascorbic acid from 20 to 150 µg/ml, preferably from 30 to 70 µg/ml, more preferably 50µg/ml.

In the present invention, the culture medium M3 and/or each of the components thereof may be of clinical grade.

In the present invention, the transduced or transformed cells culture step g) can be carried out at a temperature comprised from 36 to 38°C, or equal to 37°C.

In the present invention, the cells culture time of step g) can be comprised from 1 to 10 days, from 2 to 7 days, equal to 3 days.

In the present invention, the culture step g. can be carried out under a controlled atmosphere comprising from 5% to 10% of CO₂, for example under an atmosphere comprising at least 5% of CO₂.

In the present invention, the culture step g. can be carried out under hypoxic condition wherein the oxygen (O₂) level is from 3% to 17%, preferably from 3 to 7%, more preferably at 5%.

According to the invention, the method may comprise a further step h) of recovery of the produced iPSCs. The recovery of the produced iPSCs can be carried out in the culture medium. In the present invention, the step of recovering iPSCs can be carried out by any suitable process known to the person skilled in the art. It may be, for example, a step comprising or not one or more purification steps. It may be, for example, a process described in the Dorota Jeziorowska, Vincent Fontaine, Charlène Jouve, Eric Villard, Sébastien Dussaud, David Akbar, Valérie Letang, Pauline Cervello, Jean-Michiel Itier, Marie-Pierre Pruniaux and Jean-Sébastien Hulot, 2017. Differential Sarcomere and Electrophysiological Maturation of Human iPSC-Derived Cardiac Myocytes in Monolayer vs. Aggregation-Based Differentiation Protocols. Int. J. Mol. Sci. 18, 1173-1188.

Advantageously, the inventors have demonstrated that the obtained iPSCs from the process according to the invention have a genotype and a phenotype stable along the time and multiplication. Advantageously, the inventors have surprisingly demonstrated that the obtained iPSCs does not anarchically multiply and can be induced for proliferation and differentiation in the three embryonic sheets.

An object of the present invention is also an induced pluripotent stem cells (iPSCs) obtainable by the method of the invention. In other words, an object of the present invention is also an induced pluripotent stem cells (iPSCs) obtainable by implementing the method as defined above.

Induced pluripotent stem cells (iPSCs) obtained have the capacity of self-renewal, can differentiate in all kind of mature cells, expressing pluripotence genes for example Sox2, Nanog and Oct-4.

Advantageously, the inventors have demonstrated that the induced pluripotent stem cells (iPSC) are stable and have a balanced methylation at imprinting loci.

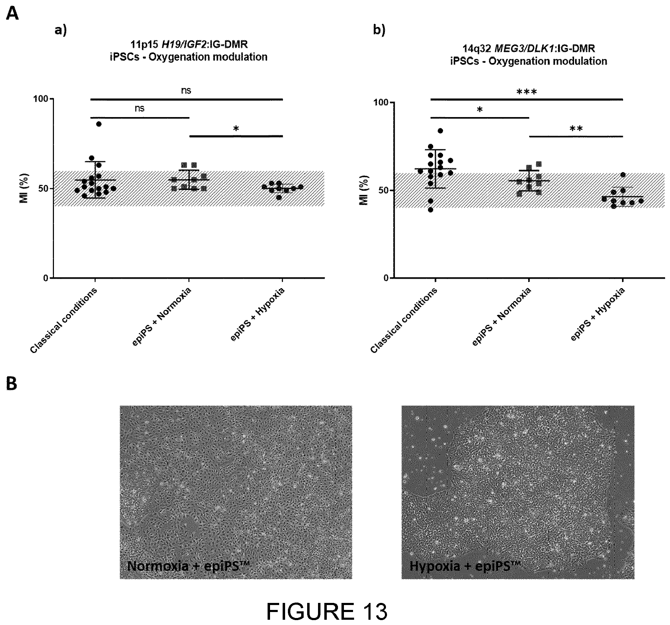

Advantageously, the inventors have demonstrated that the addition of ascorbic acid to the culture medium and culturing of the cells in hypoxic condition prevented hypermethylation of ICRs in iPSCs while preserving their capacity for proliferation and differentiation. iPSC obtained by the method of the invention present a phenotype characteristic which are classical for iPSC, a normal karyotype, expressed pluripotency markers and are able to form the three germ layers: endoderm, ectoderm and mesoderm in accordance with these cells (Figure 13).

An object of the present invention is also a kit for implementing the method according to the invention comprising a culture medium M1, a matrix gel, a culture medium M2, a culture medium M3 comprising ascorbic acid or an ascorbate or a derivative thereof.

The media M1, M2, and M3 are as defined above.

The matrix gel is as defined above.

Other advantages may further emerge to those skilled in the art on reading the examples below, illustrated by the appended figures, given by way of illustration.

### Brief description of the figures

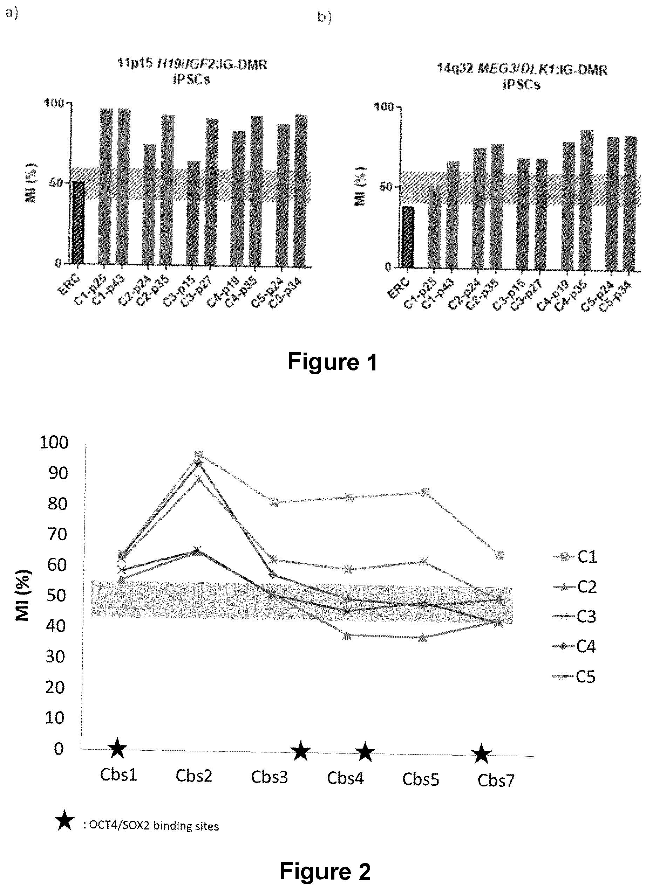

**Figure 1** represents methylation levels of five iPSC clones (C1-5) at (A) 11p15 *H19*/*IGF2*:IG-DMR (ICR1), (B) *14q32 DLK1*/*MEG3:IG-DMR* (IG-DMR), D: day, MI: methylation index, p: passage, ERC: epithelial renal cells. The ordinate represents the percentage of Methylation (MI%). C1-p25 means clone 1 passage 25, C1-p43 means clone 1 passage 43, C2-p24 means clone 2 passage 24, C2-p35 means clone 2 passage 35, C3-p15 means clone 3 passage 15, C3-p27 means clone 3 passage 27, C4-p19 means clone 4 passage 19, C5-p24 means clone 5 passage 24, C5-p34 means clone 4 passage 34.
**Figure 2** represents extensive investigation of methylation levels at the 11p15 *H19*/*IGF2*:IG-DMR (ICR1) in urine-derived iPSCs. The methylation index was studied at six CTCF binding sites. MI: methylation index, Cbs: CTCF binding site. The ordinate represents the percentage of Methylation (MI%). C1 to C5 means iPSC clones 1-5. Standard control is iPS cell from healthy human

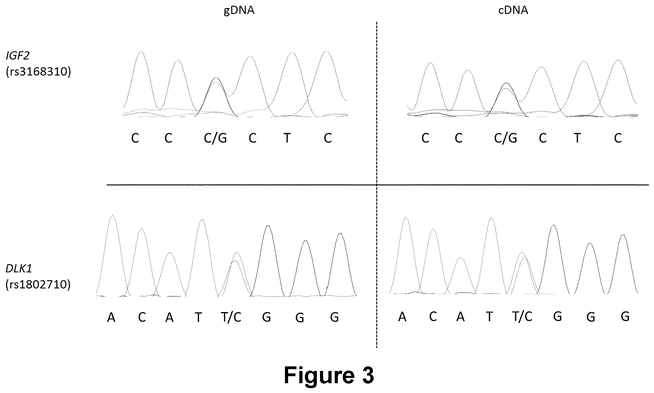

**Figure 3** represents electrophoregrams of genomic DNA (gDNA) and complementary DNA (cDNA) for one clone (Clone 1) carrying polymorphisms in *IGF2 (rs3168310)* and *DLK1 (rs1802710),* allowing the analysis of allelic-type expression of these genes.

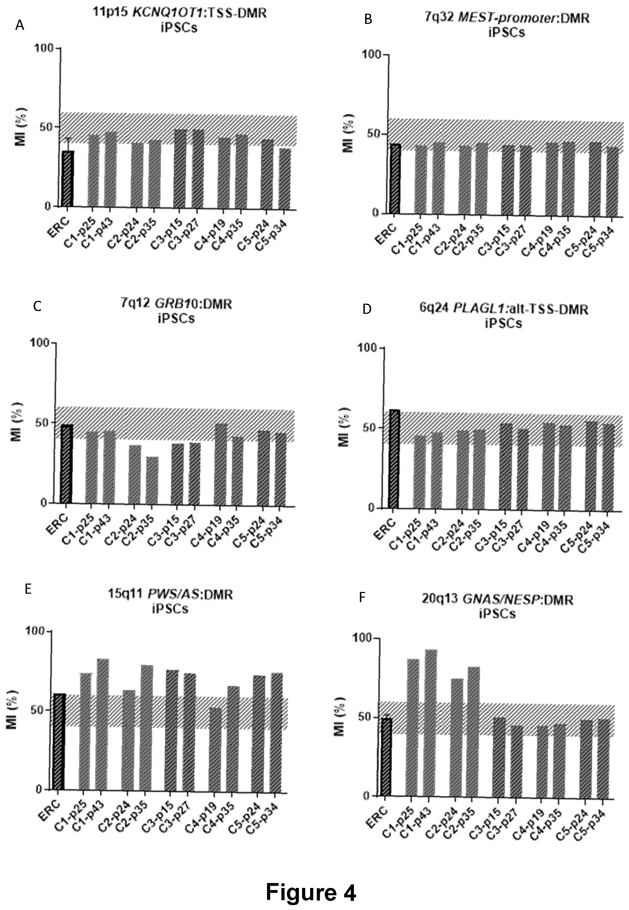

**Figure 4** represents methylation levels of five iPSC clones (C1-5) at (A) *11p15 KCNQ1OT1:TSS-DMR,* (B) *7q32 MEST promoter DMR,* (C) *7q12 GRB10:DMR,* (D) *6q24 PLAGL1:alt-TSS-DMR,* (E) *15q11 PWS*/*AS:DMR.* and (F) 20q13 *GNAS*/*NESP*:DMR. The hatched part represents the average normal MI area for each locus. MI: methylation index. p: passage, ERC: epithelial renal cells.

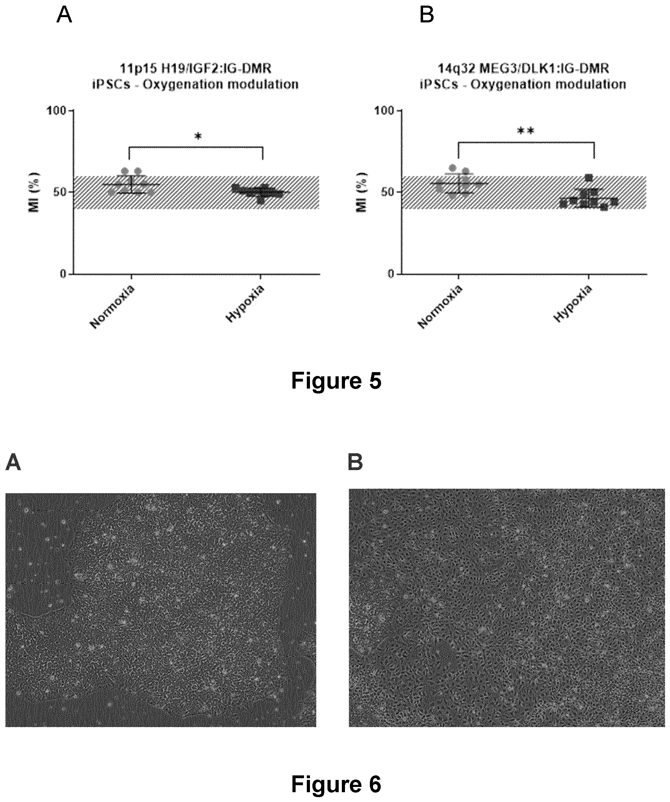

**Figure 5** represents methylation levels of nine PBMC-derived iPSC clones cultured with ascorbic acid in normoxia or hypoxia at (A) 11p15 *H19*//*GF2*:IG-DMR (ICR1) and (B) *14q32 DLK1*/*MEG3:IG-DMR.* The hatched part represents the average normal MI area for each locus. Analysis using Wilcoxon test.
**Figure 6** represents morphology of iPSC cultured with ascorbic acid addition in hypoxia (A) or normoxia (B) conditions. The same clone at passage 5, enlargement x10

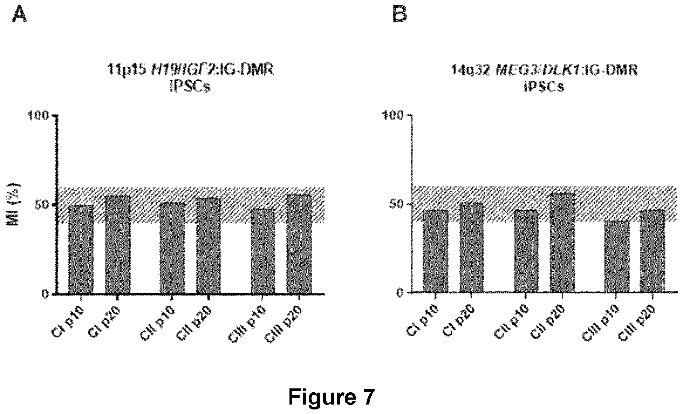

**Figure 7** represents methylation levels of three PBMC-derived iPSC clones (CI, CII, CIII) cultured with the addition of ascorbic acid to the culture medium and under hypoxia at (A) 11p15 *H19*/*IGF*2:IG-DMR (ICR1) and (B) *14q32 DLK1*/*MEG3:*/*G-DMR.* MI: methylation index, p: passage

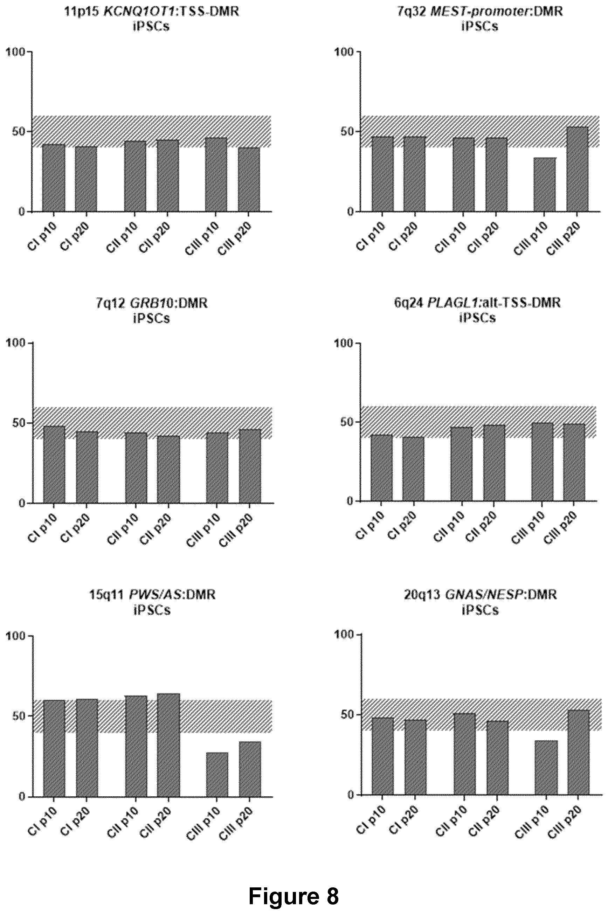

**Figure 8** represents methylation levels of three PBMC-derived iPSC clones (CI, CII, CIII) cultured under hypoxia with the addition of ascorbic acid to the culture medium at 6q24 *PLAGL*7:alt-TSS-DMR, 7q12 *GR810*:DMR, 7q32 *MEST* promoter DMR, 11p15 *KCNQ1OT1*:TSS-DMR (ICR2), 15q11 *PWS*/*AS*:DMR*,* and 20q13 *GNAS*/*NESP*:DMR*.* MI: methylation index, p: passage.
**Figure 9** represents pluripotency of iPSC control cell lines and karyotype. A. Immunostaining of iPSCs with antibodies directed against the pluripotency markers NANOG, OCT4, SOX2 (red), TRA1-60, TRA1-81, and SSEA-4 (green). Nuclei were stained with DAPI (blue). B. Normal karyotyping of cell lines. C. Positive alkaline phosphatase staining. D. RT-PCR showing the expression of the pluripotency genes of clones 7 and 14 relative to a control iPSC cell line. E. Scorecards. TM analysis assessing pluripotency and trilineage differentiation.

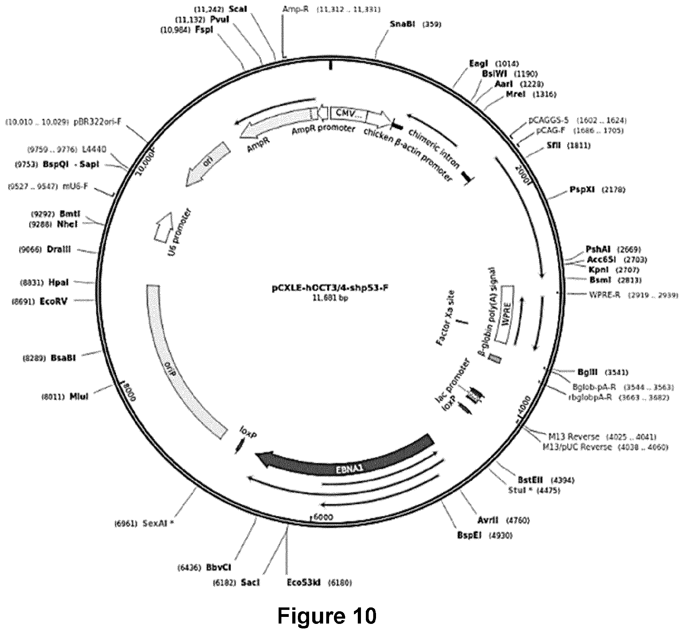

**Figure 10** represent a schema of plasmid pCXLE-hOCT3/4-shp53-F (Oct 3/4)

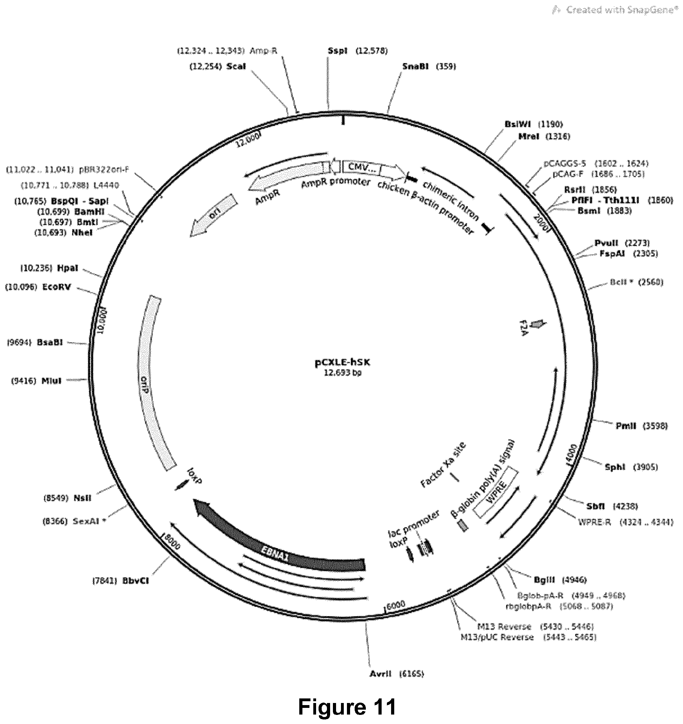

**Figure 11** represents a schema of plasmid pCXLE-hSK .

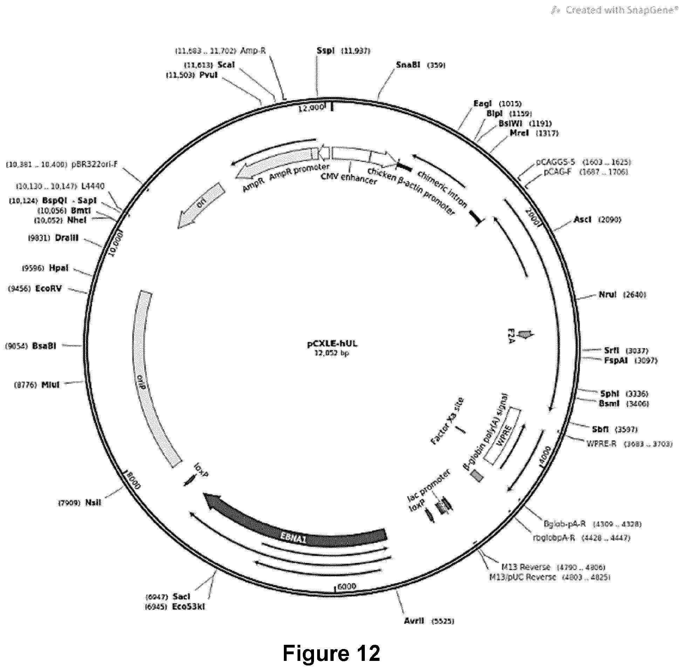

**Figure 12** represents a schema of plasmid pCXLE-hUL
**Figure 13** represents (A) Methylation levels of 16 ERC-derived iPSCs clones at passage 4 cultured in mTeSR1 medium in normoxia (classical conditions) and nine PBMC-derived iPSC clones at passage 5 (nine clones of iPSCs from one control) cultured with mTeSR1. Medium in normoxia or hypoxia at 11p15 H19/IGF2: IG-DMR (a) and 14q32 DLK1/MEG3:IG-DMR (b). The hatched part represents the average normal MI area for each locus. Analysis using Wilcoxon test and Mann-Whitney test. (B) Morphology of iPSC in hypoxia or normoxia conditions. The iPSC clone cultivated in normoxia displays a critical spontaneous differentiation unlike the same iPSC clone cultivated in hypoxia. Same clone at passage 5, enlargement x10.

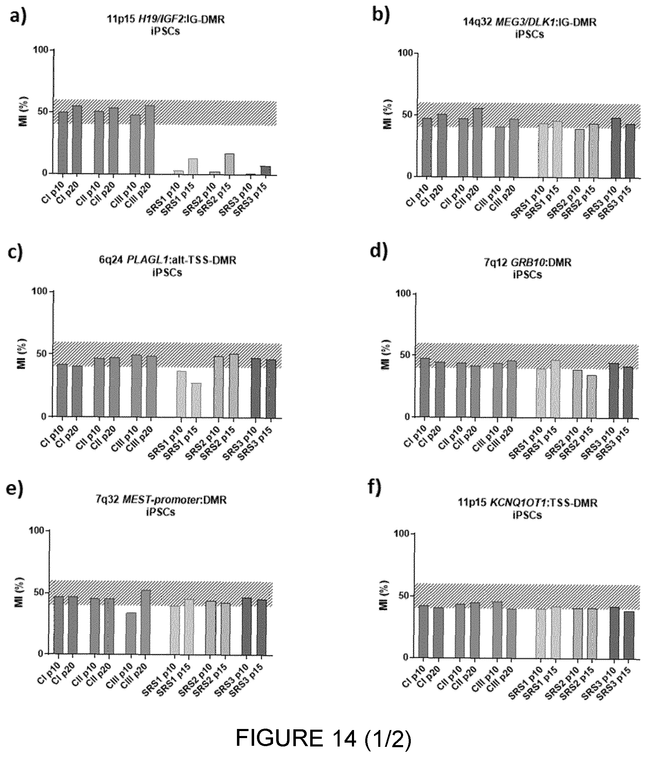

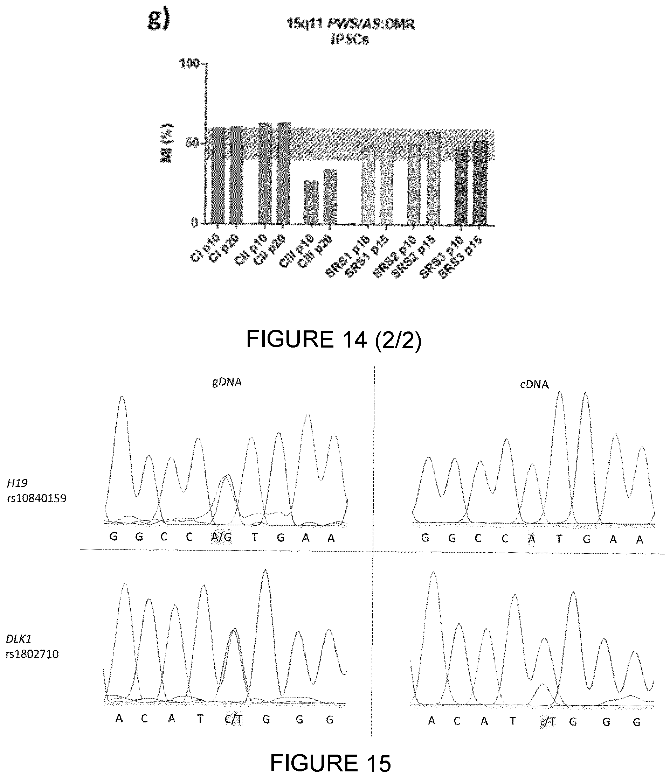

**Figure 14** represents the methylation levels of six PBMC-derived iPSC clones (Three clones of iPSCs from one control individual (CI-CIII) and from one patient with SRS (SRS1 - SRS3) cultured with the EpiPS medium and under hypoxia at (a) 11p15 H19/IGF2:IG-DMR (ICR1), (b) 14q32 DLK1/MEG3:IG-DMR, 6q24 PLAGL1:alt-TSS-DMR (c), 7q12 GRB10:DMR (d), 7q32 MEST promoter DMR (e), 11p15 KCNQ1OT1:TSS-DMR (f) and 15q11 PWS/AS:DMR. The hatched part represents the average normal MI area for each locus. MI: methylation index, p: passage
**Figure 15****.** Electrophoregrams of genomic DNA (gDNA) and complementary DNA (cDNA) for clone III cultivated in EpiPS medium in hypoxia. The clone carrying polymorphisms in *H19* (rs10840159) and *DLK1* (rs1802710), allowing the analysis of allelic-type expression of these genes.

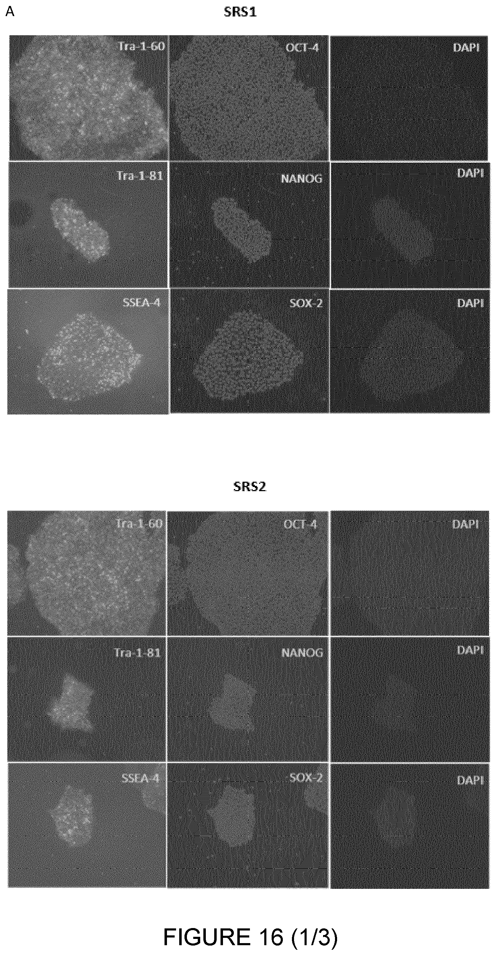

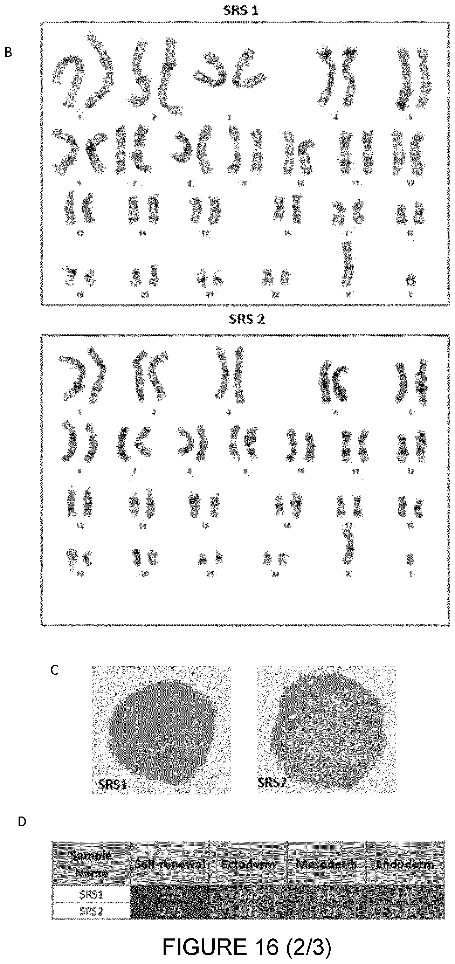

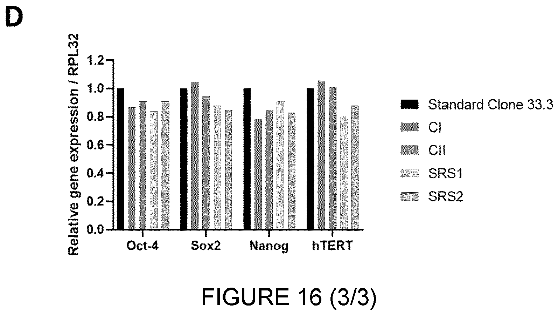

**Figure 16** represents pluripotency of iPSC from patient (SRS1, SRS2) and associated karyotypes. A. Immunostaining of iPSCs with antibodies directed against the pluripotency markers NANOG, OCT4, SOX2, TRA1-60, TRA1-81 and SSEA-4. Nuclei were stained with DAPI. B. Normal karyotyping. C. Positive alkaline phosphatase staining. D. RT-PCR showing the expression of the pluripotency genes of clones CI, CII, SRS1 and SRS2 relative to a control iPSC cell line. E. Scorecards. TM analysis assessing pluripotency and trilineage differentiation.

### EXAMPLES

### Example 1: method for producing induced pluripotent stem cells (iPSC) and study of the produced induced pluripotent stem cells (iPSC)

### I. Materials and Methods

### a) Human urine-derived iPSC reprogramming

Urine-derived iPSCs from controls were kindly provided by SFR-SANTE, iPSC core facility, INSERM, CNRS, UNIV Nantes, CHU Nantes, France. *Isolation of ERCs (epithelial renal cells) and reprogramming.*

Urine samples were collected and ERCs isolated by the iPSC core facility (INSERM, CNRS, UNIV Nantes, CHU Nantes, France) from urine samples and cultures, as previously described [36]. Briefly, cells were seeded on Matrigel-coated wells on day -1 in their usual culture media and transfected daily, from day 0 to day 10, with 625 ng of an mRNA cocktail (38% Oct4, 11.4% Sox2, 12.7% Klf4, 10.1% Lin28, 12.7% Myc, 10.1% Nanog, 5.1% nGFP, Milenyi Biotec) in Pluriton media (Reprocell, Glasgow, G20 0XA United Kingdom) supplemented with 4 ng/ml FGF2 (Peprotech, Neuilly sur Seine, France) and 200 ng/ml B18R (eBioscience, ThermoFisher scientific). From day 11, cells were cultured in Pluriton media supplemented with 4 ng/ml FGF2. Colonies were picked and expanded on feeders in KSR + FGF2 media or directly on Matrigel-coated dishes in TeSR1 or iPS Brew. The expression of pluripotency genes (*Oct4, Sox2, Nanog*) was verified by qPCR (Figure 9 and Table 7). The primers are listed in Table 7. Assays were performed to assess the ability of each iPSC clone to differentiate into the three germ line lineages [36]. The karyotype of each iPSC cell line was normal.

**Table 7. Primer sequences for pluripotent genes.**

| F: forward, R: reverse | | | | |
|---|---|---|---|---|
| Gene | Primer F | SEQ ID NO | Primer R | SEQ ID NO |
| OCT-4 | GTACTCCTCGGTCCCT TTCC | 1 | CAAAAACCCTGGCAC AAACT | 2 |
| Sox2 | TTGCTGCCTCTTTAAG ACTAGGA | 3 | CTGGGGCTCAAACTT CTCTC | 4 |
| Nano g | CTCCAACATCCTGAAC CTCAGC | 5 | CGTCACACCATTGCT ATTCTTCG | 6 |
| hTER T | CCTGGGTGGCACGGC TTTTGTTC | 7 | CAGCCTTGAAGCCGC GGTTGA | 8 |
| RPL3 2 | AGTTCCTGGTCCACAA CGTC | 9 | GTGACTCTGATGGCC AGTTG | 10 |

### iPSC culture

Human urine-derived iPSCs were initially cultured in feeder-free KSR medium (DMEM/F-12, 20% Knockout^{™} serum replacement, 1% non-essential amino acids, 1% Glutamax, 50 µM 2-mercaptoethanol, and 10 ng/ml fibroblast growth factor 2 [Peprotech Neuilly sur Seine, France]) at the iPSC core facility (INSERM, CNRS, UNIV Nantes, CHU Nantes, France). They were mechanically passaged by cutting colonies with a needle. All cells were cultured at 37°C under 20% O₂ and 5%CO₂. Then, iPSCs were cultured at the Institute of Cardiometabolism and Nutrition (ICAN, F-75013 Paris, France). Colonies were picked and expanded in mTeSR1 (Stemcell technologies, Vancouver, BC, Canada) on Matrigel matrix-coated plates. The iPSCs were passed manually once a week using a Lynx microscope (Vision Engineering, New Milford, CT, USA) and the culture medium was changed daily. The cells were cultured at 37°C under 5% CO₂ and 5% O₂.

### Human fibroblast-derived iPSCs and PBMC-derived iPSC reprogramming

Fibroblast- or PMBC-derived iPSCs were kindly provided by the Institute of Cardiometabolism and Nutrition (ICAN, F-75013 Paris, France) or the iPSC core facility (INSERM, CNRS, UNIV Nantes, CHU Nantes, France) after reprogramming by various methods (episomal vector, mRNA transfection) as previously described [36,38,39].

### Peripheral blood mononuclear cell (PBMC) culture and reprogramming with ascorbic acid

PBMCs used in the example where obtained "Établissement Français du Sang" (EFS) according to the current ethical rules: control PBMCs or where from SRS patient. Blood from SNS patients was collected after an ethical agreement. PBMCs have been recovered as described in the article Pham et al Clinical Epigenetics, 2022, 14:190. Silver-Russell syndrome is a rare condition associated with poor growth both before and after birth. Signs and symptoms vary and may include low birth weight, short stature, characteristic facial features, large head in relation to body size, body asymmetry, and feeding difficulties. It is a parental imprint disease with a methylation defect on certain imprinted genes.

After the recovery of PBMCs, they were allowed to proliferate for seven days in blood medium (StemPro34 SFM medium with 100 ng/mL Stem Cell Factor (SCF), 100 ng/mL Fms-like tyrosine kinase 3 (FLT3), 20 ng/mL Interleukin 3 (IL3), 20 ng/mL Interleukin 6 (IL6), and 2.16 U/mL erythropoietin (EPO) also mentioned Culture Medium M1. For reprogramming, 2 × 10⁵ cells were cultured in 24-well plates with a virus cocktail using the Sendai 2.0 CytoTuneiPS reprogramming kit (Life Technologies) in either 5% CO₂, 20% O₂, and 75% N₂ (normoxia condition) or 5% CO₂, 5% O₂, and 90% N₂ (hypoxia condition). The virus was removed 24 hours later (centrifugation at 300 x g for 7 min) and the cells transferred to 12-well plates in 1 ml blood media. After three days in culture, the cells were transferred to matrix gel-coated 6-well plates. At seven days post-transduction, the cells were cultured in mTeSR1 medium completed (when tested) with ascorbic acid (50µg/ml) also mentioned Culture Medium M3 and half-was renewed daily. Fifteen days post-transduction, the iPSC clones appeared, were picked, and cultured in mTeSR1 complemented with ascorbic acid (50 µg/ml).

Another example of process for obtaining iPSCs according to the invention from Peripheral blood mononuclear cell (PBMC) is also described in example 2.

### Immunofluorescence staining

iPSCs were cultured in four-well culture slides (Corning) for three days and then fixed in paraformaldehyde (4%) and permeabilized in blocking/permeabilization buffer (2% Bovine Serum Albumin (BSA), 0.5% Triton-X-100 in Phosphate Buffer Saline (PBS)) for 45 min and then incubated overnight at 4°C with primary antibodies diluted in blocking/permeabilization buffer. The cells were washed three times in PBS and incubated with Alexa-conjugated secondary antibodies and Di Aminido Phenyl Indol (DAPI), both diluted 1:1000 in blocking/permeabilization buffer, for 45 min at room temperature. The images were acquired using an epifluorescence microscope (Eclipse TE300, Nikon, Amsterdam, the Netherlands). The following antibodies were used: rabbit anti-Nanog (#4903S, 1:200, Cell Signaling-Ozyme, Beverly, MA, USA), rabbit anti-Oct4 (#3576-100, 1:200, Biovision, Cliniscience, Mountain View, CA, USA), rabbit anti-Sox2 (#AB5603, 1:200, Millipore, Ballerica, MA, USA), mouse anti-Tra-1-60 (#MAB4360, 1:100, Millipore), mouse anti-Tra-1-81 (#MAB4381, 1:100, Millipore), and mouse anti-SSEA4 (#sc-21704, 1:100, Santa Cruz, Dallas, TX, USA) (Figure 9).

### Karyotype analysis

Conventional cytogenetic studies on cell cultures at passage 20 were performed by Trousseau Hospital to verify chromosomal integrity. The process used is disclosed in Adi Baumgartner, Christy Ferlatte Hartshorne, Aris A Polyzos, Heinz-Ulrich G Weier, Jingly Fung Weier, Ben O'Brien, J Histochem Cytochem, 2018 Aug;66(8):595-606. Fifteen metaphases were analyzed (Figure 9).

### Embryoid body formation and scorecard

Generated iPSCs were grown on Matrigel, dispensed into small clumps with a cell scraper, and cultured in suspension for 10 days in TeSR-E6 medium (Stemcell). At day 8, RNA was isolated from the embryoid bodies (Ambion). A scorecard kit (Life Technologies) was used to evaluate the gene expression of the three germ layers. The scorecard plate was analysed using a StepOneplus^{™} system real time PCR (Life Technologies) (Figure 9).

### Alkaline phosphatase

Alkaline Phosphatase (AP) activity was measured using an AP detection kit (Sigma Aldrich) and performed following the manufacturer's instructions (Figure 9).

### RNA extraction and reverse transcription

RNA was extracted from iPSCs Total RNA was extracted using the NucleoSpin miRNA Kit for the isolation of small and large RNA (Macherey-Nagel, France) with DNase treatment. Both DNA and RNA were quantified using a DS-11 spectrophotometer (DeNovix). cDNA was synthesized from long RNA using the miScript PCR System (Qiagen, France) and used for quantitative PCR.

### DNA extraction

DNA was extracted from iPSCs using an in-house protocol after cell lysis by a salting-out procedure, as previously described [40,41].

### Bisulfite treatment of DNA

Sodium bisulfite treatment of DNA converts all unmethylated cytosine residues to uracil residues. The methylated cytosine residues are unaffected. This process thus generates C/T polymorphisms, which can be used to distinguish between the methylated and unmethylated allele. Genomic DNA (400 ng) was treated with sodium bisulfite using the EZ DNA Methylation Lighting kit (Zymo Research, USA), according to the manufacturer's instructions. Genomic DNA was eluted using 40 µl RNase-free H₂O and conserved at -20°C.

### TaqMan allele-specific methylated multiplex real-time quantitative PCR (ASMM RTQ-PCR) and methylation analysis.

The methylation status of seven imprinted loci (eight DMRs: 6q24 *PLAGL1*:alt-TSS-DMR, 7q12 *GR810*:DMR, 7q32 *MEST* promoter DMR, 11p15 *H19*/IGF2:IG-DMR (ICR1), 11p15 *KCNQ1OT1*-TSS-DMR (ICR2), 14q32 *MEG3*/*DLK1*:IG-DMR, 15q11 *PWS*/*AS*:DMR, 20q13 GNAS/NESP:DMR) were assessed by ASMM RTQ-PCR, as previously described [42]. The methylation index (MI) at each locus was assigned by calculating the ratio between the methylated and unmethylated alleles as follows: (amount of methylated allele/sum of both methylated and unmethylated alleles) × 100. The ASMM RTQ-PCR primers and probe sequences are disclosed in Table 8 below.

**Table 8. Primer and probe sequences for ASMM RTQ-PCR**

| F: forward, R: reverse, M: methylated allele, UM: unmethylated allele | | | | |
|---|---|---|---|---|
| **Locus** | **Primers** | **Seq ID** | **Probes** | **Seq ID** |
| Z11p15 H19/IGF2:IG-DMR | F : GTTTTTATATTAT | 11 | M : ACGTAACC CGAAACGT | 13 |
| ICR1 | AGTTTAAGTTYGT TTTAGTTGAGGT | | | |
| | R : TCCCATAAATATT CTATCCCTCACT ACC | 12 | UM : CTTACATAA CCCAAAAC ATT | 14 |
| 11p15 KCNQ1OT1:T SS-DMR ICR2 | F : GGGTTAGTTTTTT GYGTGATGTGTT T | 15 | M : ATAGCGGT CGTATTTC GATAT | 17 |
| | R : ACCTCCACACCR AAAACCCA | 16 | UM : TGTGTGAG GATAGTGG TTGT | 18 |
| 14q32 MEG3/DLK1:I G-DMR | F : AGTTTTATGTTAA GATGTTAATTATT TTTTGGA | 19 | M : TAGTTCGC GGTTCGTT AT | 21 |
| | R : ACCAAAAAACCT AACAAATCAAAA CA | 20 | UM : TAGTAGTTT GTGGTTTG TTAT | 22 |
| 7q32 MEST promoter DMR | F : GTATTAGGGTGA GATTAGGGTTAT TATGG | 23 | M : TATTACGAC GATTTCGG | 25 |
| | R : AAATATCACTCCT ACCCRCAATAAA AT | 24 | UM : AAATTTTAT TATGATGAT TTT | 26 |
| 7q12 GRB10:DMR | F : GGTTGTAATATTT AGTTTTYGAGGG ATTG | 27 | M : ACGGAGTA GCGTTCGG T | 29 |
| | R : CRTCACATAAATT TAATCCTAAAATT CCTAT | 28 | UM : ATGGAGTA GTGTTTGG TTTT | 30 |
| 15q11 PWS/AS:DMR | F : GATTTTGAGYGG TGTTTTATAGTTT AGGTT | 31 | M : AACGCATTT ACTACGAA C | 33 |
| | R : TCCTATAACCTTC CCACAACRCAC | 32 | UM : TAACACATT TACTACAAA CCA | 34 |
| 6q24 PLAGL1:alt-TSS-DMR | F : AYGGGTTGAATG ATAAATGGTAGA TGT | 35 | M : CCGTTCCG ACTCCCGA | 37 |
| | R : AACCCCAACRCC CTCCTC | 36 | UM : CACCATTC CAACTCCC AAA | 38 |
| 20q13 GNAS/AB:DM R | F : AGTAGGGGAGGT TTTGTATTTTTAT YGTA | 39 | M : TCGTAAATA ATTCGAATT CGGT | 41 |
| | R : TCCTTACCTCCA ACCACCCAC | 40 | UM: TTGGTGGG TGTGTGTT GT | 42 |
| 20q13 GNAS/NESP: DMR | F : TGTTTGGGAGAG GATAGAGGAGA | 43 | M : AGTTGCGC GTTCGGA | 45 |
| | R : ACCCCCACCTCC TTACTACACA | 44 | UM : TGTGAAGT TGTGTGTTT GGA | 46 |
| 11p15 H19/IGF2:IG-DMR CBS1 | F : GGTTTATTTTGTT GATTTTATTAAGG GAG | 47 | M : ATGGCGTA GAATCGGT TGTACGT | 49 |
| | R : TTTCTATAAATAA ACCCCAACCAAA C | 48 | UM : TTAGAAGT GGTTGTGT GGTGGTAG T | 50 |
| 11p15 H19/IGF2:IG-DMR CBS3 | F : GGATGGTATAGA ATYGGTTGTAAG TGTG | 51 | M : AAAAGTGG TCGCGCGG CG | 53 |
| | R : AACCCCATCCAA AAAAAACTTAAAC | 52 | UM : AAAGTGGT TGTGTGGT GGTAGT | 54 |
| 11p15 H19/IGF2:IG-DMR CBS4 | F : GGATGGTGTAGA ATTGGTTGTAGT TGT | 55 | M : TACGAAAC CCGAAACG T | 57 |
| | R : TCCCATAAATATC CTATCCCTAATAA CC | 56 | UM : ACTTACAAA ACCCAAAA CAT | 58 |
| 11p15 H19/IGF2:IG-DMR CBS5 | F : GTTTGGATGATT TGGGATGTTTTT A | 59 | M : ATAACTAC GCGACGAC AA | 61 |
| | R : TCTAAAAACTTCC CCTTCAATCTCA | 60 | UM : TAACTACAC | 62 |
| | | | AACAACAAT AC | |
| 11p15 H19/IGF2:IG-DMR CBS7 | F : TGGGTTTTGGGT GTTGTTTTTAG | 63 | M : ACGGAAAT GGTCGAGA GGC | 65 |
| | R : CAAAACAAACCC CCACATTTTAC | 64 | UM : AGATGGAA ATGGTTGA GAGGTGGT A | 66 |

### gDNA and cDNA sequencing

To assess the mono or biallelic expression of imprinted genes, two single nucleotide polymorphisms from the *H19* (rs217727) and *DLK1* (rs1802710) gDNA and cDNA were sequenced by standard Sanger sequencing by Eurofins Genomics (Germany). The primers used for gDNA and cDNA amplification and sequencing are disclosed in Table 9 below. The sequencing products were then analyzed using Sequencing Analysis 5.2.

**Table 9. Primers used for gDNA and cDNA amplification and sequencing**

| F: forward, R: reverse | | | |
|---|---|---|---|
| **Primer** | **5' Sequence 3'** | **Application** | **SEQ ID NO** |
| H19-ex1F | CAGTCACCCGGCCCAGAT | gDNA | 67 |
| H19-ex1R | AAGACACCATCGGAACAGCA | gDNA | 68 |
| H19-ex4-5R(exon-exon junction) | GCTCTGGAAGGTGAAGCTAG | cDNA | 69 |
| H19-ex5-F2 | AGGCCGTCTCCACAACTCC | cDNA | 70 |
| DLK1-ex5-F | TGCGTGGATGATGAGGGC | gDNA | 71 |
| DLK1-ex5R | CTTGCACAGACACTCGTAGC | gDNA/cDNA | 72 |
| DLK1-ex4-5F (exon-exon junction) | TGATCAACGGCTCCCCCTGC | cDNA | 73 |

### Statistics

Data in the figures are presented as means. All graphs were generated and statistical analysis performed using GraphPad Prism 6 (USA) or Excel.

### II. RESULTS

### Methylation of ICRs in urine-derived iPSCs and during chondrogenic differentiation

The methylation levels was studied at eight imprinted loci in epithelial renal cells (ERCs) in five available controls urine-derived iPSC clones (C1-5) at various passages of cell culture. Certain iPSC clones showed hypermethylation at loci *H19*/*IGF2*:IG-DMR (ICR1), 14q32 *MEG3*/*DLK1*:IG-DMR, 15q11 *PWS*/*AS*:DMR (figure 1a, 1b, 4E) and 20q13 *GNAS*/*NESP*:DMR (Figure 4 F). Methylation was normal for all the urine-derived iPSCs at 6q24 *PLAGL1*:alt-TSS-DMR, 7q12 *GRB10*:DMR, the 7q32 *MEST* promoter DMR, and 11p15 *KCNQ1OT1*:TSS-DMR (ICR2) (Figure 4 A - D). No clone showed balanced methylation at all loci (Figure 1 and Figure 4).

### Extensive analysis of the IGF2/H19 imprinting control region

The methylation profile of the *H19*/*IGF2*:IG-DMR (ICR1) domain at six CTCF binding sites (CBS 1, 2, 3, 4, 5, 7) in the urine-derived iPSCs clones before chondrogenic differentiation was studied (Figure 2). One clone (C1) was hypermethylated at all CBSs and the others were almost all normal at all CBSs, except at CBS2, for which all clones showed an abnormal and higher level of methylation.

### Imprint relaxation

Polymorphisms in *IGF2* (rs3168310) and *DLK1* (rs1802710) in the genomic DNA of C1 was identified, allowing to study the mono or biallelic expression of these imprinted genes. Both *IGF2* and *DLK1* showed biallelic expression (Figure 3), indicating a loss of imprinting (LOI) at these two loci, results in accordance with the hypermethylation of the corresponding ICRs.

### Impact of the method and cell type on iPSC reprogramming

The methylation levels at *H19*/*IGF2:*IG-DMR (ICR1) in 10 iPSC clones from different somatic cells of origin reprogrammed by various methods and cultured in feeder-free medium, in particular mTeSR1 or KSR feeder-free medium, were studied. All iPSC clones showed aberrant hypermethylation at 11p15 ICR1, independently of the reprogramming method (mRNA transfection or episomal vector) or somatic cell of origin (skin fibroblast or peripheral blood mononuclear cells-PBMCs) (Table 10).

**Table 10. Methylation index at 11p15 H19/IGF2:IG-DMR (ICR1) of 10 iPSC clones (C6-C15). C6-C12 were reprogrammed from skin fibroblasts and C13-C15 from peripheral blood mononuclear cells (PBMCs). The reprogramming of somatic cells into iPSCs was carried out using mRNA transfection (C6-C11) or an episomal vector (C12-C15).**

| **Clone** | **Somatic cells of origin** | **Technic of reprogramming** | **11p15 ICR1 MI (%)** |
|---|---|---|---|
| C6 | Skin fibroblast | mRNA transfection | 96 |
| C7 | Skin fibroblast | mRNA transfection | 80 |
| C8 | Skin fibroblast | mRNA transfection | 70 |
| C9 | Skin fibroblast | mRNA transfection | 68 |
| C10 | Skin fibroblast | mRNA transfection | 89 |
| C11 | Skin fibroblast | mRNA transfection | 66 |
| C12 | Skin fibroblast | Episomal vector | 67 |
| C13 | PBMC | Episomal vector | 64 |
| C14 | PBMC | Episomal vector | 67 |
| C15 | PBMC | Episomal vector | 68 |

### Impact of culture conditions on methylation at imprinted loci

As similar hypermethylation was observed independently of the cell type of origin and reprogramming method, the culture conditions was suspected to have a major influence. This hypothesis was tested by adding ascorbic acid (50 µg/mL) to a culture medium for iPSCs (mTeSR1) and culturing the iPSCs in hypoxia or normoxia conditions. The reprogramming experiments from PBMCs was carried out, which are easier to obtain, cultivate, and amplify than ERCs.

First, the effect of oxygenation on methylation levels at 11p15 H19/IGF2:IG-DMR (ICR1) and 14q32 MEG3/DLK1:IG-DMR loci on nine pairs of iPSCs clones cultivated under either hypoxia or normoxia with ascorbic acid was tested (Figure 5). Some clones cultivated in normoxia and ascorbic acid display a slight hypermethylation at H19/IGF2:IG-DMR (ICR1) and 14q32 MEG3/DLK1:IG-DMR. The experiment did not exceed passage 5 due to spontaneous differentiation occurring in clones cultivated in normoxia and vitamin C (Figure 6). These results clearly demonstrate the synergistic effect of hypoxia and ascorbic acid on the maintenance of pluripotency and balanced methylation. In a second time, three clones were cultured in hypoxia until 20 passages and IM was measured at 11p15 *H19*/*IGF2:IG-DMR* and 14q32 *MEG3*/*DLK1:*IG-DMR (Figure 7), as well as in six other imprinted loci (Figure 8). For the major part of loci studied, the methylation stayed between 45- 60% and remained stable from the passage 10 to 20. The imprinted loci 15q11 *PWS*/*AS*:DMR seems to be at the limit of the hypermethylation for the clone I at p10 and displays a slight hypermethylation in the clone I at p20, II at p10 and II at p20. Surprisingly, the clone III at p10, displays loss of methylation at 7q32 *MEST* promoter DMR, 20q13 *GNAS*/*NESP*:DMR and 15q11 *PWS*/*AS*:DMR loci; this hypomethylation is corrected at passage 20 except for the locus 15q11 *PWS*/*AS*:DMR in clone III.

The effect of ascorbic acid on methylation levels at 11p15 *H19*/*IGF2*:IG-DMR (ICR1) and 14q32 *MEG3*/*DLK1*:IG-DMR loci on sixteen iPSCs clones cultivated under normoxia in mTeSR1 medium and on nine clones cultivated in mTeSR1 medium comprising 50 mg/mL of ascorbic acid (culture medium M3) in normoxia. In conventional culture conditions, most of iPSCs clones displayed an hypermethylation in 14q32 *MEG3*/*DLK1:IG-DMR* unlike iPSCs cultivated in medium M3 in normoxia (Figure 13A). To the contrary, iPSCs cultivated in cultivated in medium M3 in normoxia exhibited a frequent and complete spontaneous differentiation (Figure 13B). The high concentration of ascorbic acid is suspected to play a major role in this phenomenon since ascorbic acid is a well-known differentiating factor. The same 9 clones in culture medium M3 under hypoxia were cultured. iPSCs clones cultivated in culture medium M3 in hypoxia showed both balanced methylation at 11p15 *H19*/*IGF2:*IG-DMR and 14q32 *MEG3*/*DLK1:IG-DMR* loci and optimal pluripotency status without frequent spontaneous differentiation (Figure 13A and 13B).

These results clearly demonstrate an unexpected and synergistic effect of hypoxia and culture medium M3 (mTeSR1 medium comprising 50 mg/mL of ascorbic acid) on the maintenance of pluripotency and balanced methylation of iPSCs.

Additionally, three clones (CI-CIII) were cultured in hypoxia until 20 passages and IM was measured at 11p15 *H19*/*IGF2*:IG-DMR and 14q32 *MEG3*/*DLK1*:IG-DMR (Figures 14a and 14b), as well as in five other imprinted loci (Figures 14c to 14g). For the major part of loci studied, the methylation stayed between 45-60% and remained stable from the passage 10 to 20. The imprinted loci 15q11 *PWS*/*AS*:DMR seems to be at the limit of the hypermethylation for the clone I at p10 and displays a slight hypermethylation in the clone I at p20, II at p10 and II at p20. Surprisingly, the clone III at p10, displays loss of methylation at 7q32 *MEST* promoter DMR, and 15q11 *FWS*/*AS*:DMR loci; this hypomethylation is corrected at passage 20 except for the locus 15q11 *PWS*/*AS*:DMR in clone III. After identifying polymorphisms in the imprinted genes *H19* (rs10840159) and *DLK1* (rs1802710) in clone III, the allelic expression of these genes. *H19* and *DLK1* was studied and showed a mono-allelic expression corresponding to a maintenance of the parental imprinting (Figure 15).

A second reprogramming, i.e. obtaining iPSC from PBMCs, was carried out on another PBMC control under the determined culture conditions. All results are in accordance with those obtain for the first reprogrammation.

Another reprogramming, i.e. obtaining iPSC from PBMCs, was carried out on PBMCs from a SRS patient (SRS1 - SRS3) with a heterozygous deletion of the paternal *H19*/*IGF2*:IG-DMR region. This is an isolated genetic anomaly, so the other loci are not affected. The measure of the methylation levels was carried out on different loci and the obtained results are represented on figure 14. This three patient's iPSCs clones (SRS1-3) shown loss of methylation at the 11p15 *H19*/*IGF2:*IG-DMR and balanced methylation at the 14q32 *MEG3*/*DLK1*:IG-DMR at passage 10 and 15 (Figures 14a and 14b). All other loci show balanced methylation for all three clones except for the 6q24 *PLAGL1*:alt-TSS-DMR locus where one clones exhibits a loss of methylation. (Figures 14c to 14g). The allelic expression was not performed due to the absence of polymorphism in *IGF2, H19, DLK1* imprinted genes in this patient.

In conclusion, the results clearly demonstrate that the use of ascorbic acid with hypoxia culture, allows to obtain iPSCs with a balanced methylation at the imprinted loci and an optimal pluripotency status.

These results clearly confirm that the method according to the invention allows obtaining iPSCs with a balanced methylation at the imprinted loci and an optimal pluripotency status.

In other words, the methylation levels at eight imprinted loci in iPSCs at various passages of cell culture, was assessed. As demonstrated, abnormal methylation levels were found, with hypermethylation at 11p15 H19/IGF2:IG-DMR and 14q32 MEG3/DLK1:IG-DMR, independently of the reprogramming method and cells of origin. Hypermethylation at these two loci led to the loss of parental imprinting (LOI), with biallelic expression of the imprinted genes IGF2 and DLK1, respectively. The addition of ascorbic acid (50 µg/mL) to the culture medium combined with culturing of the cells under hypoxic conditions prevented hypermethylation at *H19*/*IGF2:*IG-DMR (ICR1) and 14q32 MEG3/DLK1:IG-DMR, as well as at other imprinted loci, while preserving the proliferation and pluripotency qualities of these iPSCs.

In conclusion, the obtained results clearly demonstrate that the use of ascorbic acid with hypoxia culture, allows to obtain iPSCs with a globally balanced methylation at the imprinted loci and an optimal pluripotency status. These results clearly confirm that the method according to the invention allows obtaining iPSCs with a globally balanced methylation at the imprinted loci and an optimal pluripotency status.

### III. DISCUSSION

With the goal of developing a cellular model of IDs, an extensive quantitative analysis of methylation levels of several ICRs was performed to assess whether parental imprinting is maintained during the reprogramming and culture of human iPSCs. Abnormal methylation levels was found at the two imprinted loci governed by a paternally methylated DMR in human feeder-free iPSCs derived from controls with no IDs, independently of the reprogramming method or somatic cell of origin. Hypermethylation at 11p15 *H19*/*IGF2:*IG-DMR and 14q32 *MEG3*/*DLK1:IG-DMR* led to the loss of parental imprinting, with biallelic expression of the imprinted genes *IGF2* and *DLK1,* respectively. The method for obtaining the iPSCs according to the invention comprise a culture medium comprising ascorbic acid combined with culture in hypoxia. This surprisingly allows to obtain iPSCs from PBMCs without the aberrant methylation, in particular at these two ICRs. Analysis of the methylation of eight ICRs showed that the process of the invention comprising these particular culture conditions allow surprisingly to prevent the hypermethylation of ICRs in iPSCs generated from both control and SRS patient PBMC while retaining the qualities of proliferation and pluripotency and, thus, offering a human cellular model of ID.

The results and those of several other previous studies suggest a trend towards hypermethylation of certain ICRs in human iPSCs when cultured, especially paternally methylated ICRs, and LOI of certain imprinted genes. Several groups have reported aberrant DNA methylation or LOI at 11p15 *H19*/*IGF2:*IG-DMR and 14q32 *MEG3*/*DLK1:IG-DMR* in mouse and human pluripotent stem cells, which persisted after differentiation into various cell types, but they focused exclusively on these two loci [10,15-17]. These and several other loci were tested and it was found that imprinted genes governed by a paternally methylated DMRs appear to be more frequently affected by LOI in iPSCs than those controlled by a maternally methylated DMR [10]. Bar and Benvenisty hypothesized that paternally imprinted methylated regions are more sensitive to aberrations during reprogramming due to different mechanisms for protecting imprinted regions from demethylation and *de novo* methylation in paternally versus maternally methylated regions [10]. The methylation profile at the *H19*/*IGF2:*IG-DMR (ICR1) domain at six CBSs (CTCF binding sites) was studied and it was found the highest level of methylation in the most distal CBS to the OCT4 and SOX2 binding sites (CBS2), which are known to protect the maternal allele against *de novo* methylation during early embryogenesis [18,19], consistent with the hypothesis of Bar and Benvenisty. On the other hand, several groups have suggested that clones with aberrant hypermethylation are selected during the culture of iPSCs due to the upregulation of growth-promoting genes, such as *IGF2,* or the silencing of growth-restricting genes, which promote self-renewal, growth, and survival of iPSCs in culture [20,21].

In contrast to embryonic stem cells (ESCs), global hypermethylation of gDNA of the entire genome has been described in iPSCs at early passages (i.e., p3-p5), which then disappeared during subsequent passages [22-24]. By contrast, it was found that abnormal methylation in ICRs persisted during *in vitro* culture up to 43 passages of iPSCs. Recent studies in mouse and human iPSCs have suggested that DNA methylation of the genome in these cells is highly dynamic, cycling between *de novo* methylation and its erasure [25,26]. However, such turnover was not observed in imprinted regions, which is consistent with the results [26].

Several iPSC models derived from patients with IDs have been described in the literature. For example, Chamberlain and Burnett derived iPSCs from patients with Prader-Willi syndrome due to a paternally inherited deletion of chromosome 15q11-q13 and Chang et al. derived iPSCs from patients with Beckwith-Wiedemann syndrome with paternal uniparental disomy of chromosome 11. In these studies, human iPSCs derived from controls and patients showed the same methylation patterns as the fibroblast lines from which they were derived [6-8]. However, the authors studied neither methylation levels in patient and control iPSCs in other ICRs nor mono- or biallelic expression of imprinted genes, and their results are not in accordance with those of other studies. In particular, these authors studied methylation levels at only one locus for Chamberlain (15q11 PWS/AS:DMR) and two loci for Chang (H19/IGF2:IG-DMR and 11p15 KCNQ1OT1:TSS-DMR) which explain why they did not observe the methylation abnormalities specific to reprogramming and culture of iPSCs under conventional conditions Indeed, Okuno et al. demonstrated that a fully methylated status for chromosome 15q11 in fibroblasts could be reversed to a partially unmethylated status in at least some of the iPSCs after reprogramming [27], and Nazor et al. demonstrated a loss of methylation at the 15q11 locus in a subset of control iPSC lines [28]. As imprinted genes are co-regulated and organized in an imprinted-gene network (IGN), abnormal methylation at one locus can modify the expression of imprinted genes of other imprinted loci, even if the methylation of the corresponding ICR is not affected but also the expression of non-imprinted genes [4,29-33]. This is why the level of methylation at all ICRs has to be studied in iPSCs derived from patients with IDs.

As demonstrated, the method of the invention comprising a culture medium comprising ascorbic acid (medium M3) during the culture of human iPSCs and under hypoxia, allows surprisingly and unexpectedly to avoid aberrant methylation of ICRs and retained proliferation and pluripotency qualities of iPSCs. The extensive and quantitative analysis of ICR methylation levels in iPSCs derived from controls and cultured with the addition of ascorbic acid and under hypoxia showed globally balanced methylation at imprinted loci tested, implying, moreover, a synergistic effect of these two elements. Indeed, in normoxia in presence of ascorbic acid, not only some clones acquire a light but significatively hypermethylation during the passages but also iPSCs lose their ability to proliferate and differentiate spontaneously.

Here, it is reported, for the first time, an extensive analysis of methylation levels of several ICRs involved in human IDs in iPSCs. In addition, the results clearly demonstrate that the process of the invention allows to obtain iPSCs from PBMCs, the obtained iPSCs having

### CONCLUSIONS

Through an extensive and quantitative analysis of the methylation levels of ICRs in iPSCs, it has been found hypermethylation of certain ICRs in human iPSCs, particularly paternally methylated ICRs, and subsequent LOI of certain imprinted genes. The method of obtaining iPSCs comprising ascorbic acid during the culture of iPSCs under hypoxia prevented the hypermethylation of ICRs and allows surprisingly and unexpectedly to maintain balanced methylation at imprinted loci and pluripotency of iPSCs. The results clearly demonstrate a synergistic effect of ascorbic acid and hypoxic conditions in the culture method for obtaining Induced pluripotent stem cells (iPSC). As balanced methylation is maintained during the reprogramming and culture of iPSCs in these conditions, human iPSCs are a promising cellular model to study the physiopathology of IDs and test therapies, after differentiation, in tissues of interest.

### Example 2: method for producing induced pluripotent stem cells (iPSC)

### MATERIALS AND METHODS

### 1. MATERIALS

- Stem-Pro-34^{®} SFM medium: Stem-Pro-34^{®} SFM basal Medium + Stem-Pro-34^{®} Supplement (Invitrogen, ref : 10639-011)
- Medium mTeSR1^{®} (StemCell, ref : 05850)
- Medium DMEM/F12 (Invitrogen, ref : 11330-057)
- Matrigel grade hESC/hiPS (Corning, ref : 354277)
- Recombinant human SCF (Peprotech, ref : 300-07)
- Recombinant human IL-3 (Peprotech, ref : 200-03)
- Recombinant human IL-6 (Invitrogen, ref: PHC0065)
- Recombinant human EPO (Mililpore, ref : 329871-50µg)
- Recombinant human FLT-3 (Invitrogen, ref: PHC9414)
- Ascorbic acid (Sigma, ref: A4403)
- PBS 1X without calcium and magnesium (Invitrogen, ref : 20012-019)
- Cytotune Sandai 2.0 (Invitrogen, ref: A16517)
- 6 wells plates coated with matrigel grade hESC/hiPS
- 12 and 24 wells plates
- Falcons 15 mL, 50mL
- Organ cell (Falcon, ref : 353037)
- Pipettes 5, 10 and 25 mL
- Micropipettes 100 and 1000µL
- Tips 300µL (Dutscher, ref : 130930T)
- Tips 100 et 1000µL
- Vacuum pump
- Centrifuge
- Microscope
- Countess^{™} II, counting slides and trypan blue (Invitrogen)
- Class II microbiological safety station
- Incubator 37°C, 5% CO₂, 5% O₂.

### 2. METHODS

### 2.1. MEDIUM PREPARATION

### 2.1.1 MEDIUM « BLOOD MEDIA » (CULTURE MEDIUM M1):

| **Composants** | **Volumes** |
|---|---|
| Stem-Pro-34^{®} SFM Medium complete | 50 mL |
| SCF (100 µg/mL) | 50 µL |
| FLT3 (100 µg/mL) | 50 µL |
| IL3 (100 µg/mL) | 10 µL |
| IL6 (100 µg/mL) | 10 µL |
| EPO (54 U/µL) | 2 µL |

### 2.1.2. MEDIUM « BLOOD BASAL MEDIA » (CULTURE MEDIUM M2):

| **Composants** | **Volumes** |
|---|---|
| Stem-Pro-34^{®} SFM basal Medium | 50 mL |
| Stem-Pro-34^{®} Supplement | 1.25mL |

### 2.1.3. METHYLATION MAINTENANCE MEDIUM (CULTURE MEDIUM M3):

| **Composants** | **Volumes** |
|---|---|
| mTeSR1 complete | 50 mL |
| Ascorbic acid (50 mg/mL) | 50 µL |

### 2.2. PBMC culture:

### 2.2.1. Culture of fresh PBMC :

- Recovering PBMC after separation
- Counting PBMC in Countess^{™} II
- Collect 1 million cells and place them in a 12-well plate pre-filled with 2mL of blood media (CULTURE MEDIUM M1).
- Place in a 5% CO₂ incubator at 37°C.
- Change the medium every other day by replacing half of the medium with 1mL of Blood media for 6-8 days.

### 2.2.2 Culture of freezed PBMC :

- Recover PBMC cryotubes (1million cells/cryotube) in the -150°C freezer and transfer to ice.
- Using a 10 mL pipette, collect 9 mL of blood basal media and transfer to a 15mL falcon.
- Put the cryotube in the oven at 37°C for 3 min.
- Under PSM, recover the contents of the cryotube with a P1000 pipette and transfer it to the 15mL falcon containing the 9mL of blood basal media.
- Centrifuge the cells at 300g for 7 min.
- Aspirate the supernatant and resuspend the pellet in 2mL of Blood media.
- Place the cells in a 12-well plate
- Place in a 5% CO₂ incubator at 37°C.
- Change half of the medium every other day for 6-8 days before transduction with Sendai virus by adding « blood media ».

### 2.3. PREPARATION AND CELLS TRANSDUCTION (DAY 0)

### 2.3.1. PREPARATION OF DIFFERENT REAGENTS:

This procedure describes the different reagents to be prepared for a 24-well culture plate, the quantities of the different components will be adapted according to the number of plates.
- Remove the 3 Sendai virus tubes and put them on ice.
- In an eppendorf tube, prepare the virus mix: refer to the certificate of analysis (CoA) provided with the kit for the titration and the recommended volumes of each virus, dilute at 1/5 the viruses compared to the titration and the recommended volume of the certificate of analysis.
- Keep the eppendorf tube on ice until use.

### 2.3.2. PBMC TRANSDUCTION

- Collect and transfer PBMC to a 1.5 mL eppendorf tube
- Centrifuge at 300g for 7 min, then aspirate the supernatant
- Resuspend the cell pellet in 500µL of blood media and add the cocktail of viruses coding for Oct3/4, Sox2, Klf4, L-Myc.
- Then seed 2×10⁵ cells per well of 24-well plate.
- Put the 24-well plate in the incubator 5% CO₂, 5% O₂, 90% N₂ at 37°C (hypoxic condition).
- The next day, remove the virus by centrifuging the PBMC at 300g for 7 min.
- Resuspend PBMC in 1 mL of blood media and transfer to a 12-well plate.
- Put the 12-well plate in the incubator 5% CO₂, 5% O₂, 90% N₂ at 37°C 2.3.3.PBMC CULTURE POST INFECTION
- Let PBMC grow for 3 days in suspension in blood media.
- Prepare 2 wells of 6-well matrigel coated plates
- Three days post-infection, collect PBMC in a 1.5 mL eppendorf, centrifuge them at 300g for 7 min and resuspend them in 0.5 mL of « basal media » (CULTURE MEDIUM M2).
- Aspirate the matrigel present in the 2 wells of the 6-well culture plate.
- Transfer the PBMC suspension to the two wells of the 6-well plate at a rate of 0.5 mL per well and then complete to 1 mL with « basal media ».
- Put the 6-well plate in the hypoxia incubator 5% CO₂, 5% O₂, 90% N₂ at 37°C.
- Observe the cells over 3 days without changing the medium. The cells in suspension adhere to the bottom of the well.
- At day 7 post transduction, aspirate the blood media and put 1.5mL of mTeSR1@ + ascorbic acid at 50µg/ml (CULTURE MEDIUM M3) in the wells every day.
- Watch the cells daily to check for proliferation and change in cell morphology.
- Around day 15 post transduction, iPS colonies start to appear.
- Colonies are collected with a 10µL tip and transferred to 4-well plates coated with matrigel containing 500µL of mTeSR1@ + ascorbic acid at 50µg/ml + 10µM of Rock Inhibitor (P1).
- The next day, change the medium with 500µL of mTeSR1@ + ascorbic acid at 50µg/ml and put the plate back in the incubator.
- Change the medium every day with 50µL of mTeSR1@ supplemented with 50µg/ml ascorbic acid.
- After 7-10 days, pass the iPS in a B3.5 coated with matrigel (P2)
- After 4-7 days, pass the iPS in a B6 coated with matrigel (P3)
- After 4-7 days, pass the iPS in a B6 coated with matrigel (P4)
- The iPS cells are kept in hypoxia (5% oxygen) permanently.
- Freeze iPS at passage 5 and 10 for each clone.
- Then perform freezing every 5 passages of iPS.

Wait for at least passage 15 to perform clone banking and to start differentiations on iPS.

### List of references

1. DeChiara TM, Robertson EJ, Efstratiadis A. Parental imprinting of the mouse insulin-like growth factor II gene. Cell. 1991 Feb 22;64(4):849-59.
2. Eggermann T, Perez de Nanclares G, Maher ER, Temple IK, Tümer Z, Monk D, et al. Imprinting disorders: a group of congenital disorders with overlapping patterns of molecular changes affecting imprinted loci. Clin Epigenetics. 2015;7:123.
3. Wakeling EL, Brioude F, Lokulo-Sodipe O, O'Connell SM, Salem J, Bliek J, et al. Diagnosis and management of Silver-Russell syndrome: first international consensus statement. Nat Rev Endocrinol. 2017;13(2):105-24.
4. Abi Habib W, Brioude F, Azzi S, Rossignol S, Linglart A, Sobrier M-L, et al. Transcriptional profiling at the DLK1/MEG3 domain explains clinical overlap between imprinting disorders. Sci Adv. 2019 Feb;5(2):eaau9425.
5. Abi Habib W, Brioude F, Azzi S, Rossignol S, Linglart A, Sobrier M-L, et al. Transcriptional profiling at the DLK1/MEG3 domain explains clinical overlap between imprinting disorders. Sci Adv. 2019;5(2):eaau9425.
6. Burnett LC, LeDuc CA, Sulsona CR, Paull D, Eddiry S, Levy B, et al. Induced pluripotent stem cells (iPSC) created from skin fibroblasts of patients with Prader-Willi syndrome (PWS) retain the molecular signature of PWS. Stem Cell Res. 2016;17(3):526-30.
7. Chamberlain SJ, Chen P-F, Ng KY, Bourgois-Rocha F, Lemtiri-Chlieh F, Levine ES, et al. Induced pluripotent stem cell models of the genomic imprinting disorders Angelman and Prader-Willi syndromes. Proc Natl Acad Sci USA. 2010 Oct 12;107(41):17668-73.
8. Chang S, Hur SK, Naveh NSS, Thorvaldsen JL, French DL, Gagne AL, et al. Derivation and investigation of the first human cell-based model of Beckwith-Wiedemann syndrome. Epigenetics. 2020 Dec 29;1-11.
9. Grybek V, Aubry L, Maupetit-Méhouas S, Le Stunff C, Denis C, Girard M, et al. Methylation and transcripts expression at the imprinted GNAS locus in human embryonic and induced pluripotent stem cells and their derivatives. Stem Cell Reports. 2014 Sep 9;3(3):432-43.
10. Bar S, Benvenisty N. Epigenetic aberrations in human pluripotent stem cells. EMBO J. 2019 17;38(12).
11. Chamberlain SJ, Li X-J, Lalande M. Induced pluripotent stem (iPS) cells as in vitro models of human neurogenetic disorders. Neurogenetics. 2008 Oct;9(4):227-35.
12. Yamanaka S. Induced pluripotent stem cells: past, present, and future. Cell Stem Cell. 2012 Jun 14;10(6):678-84.
13. Germain ND, Levine ES, Chamberlain SJ. IPSC Models of Chromosome 15Q Imprinting Disorders: From Disease Modeling to Therapeutic Strategies. Adv Neurobiol. 2020;25:55-77.
14. Sabitha KR, Shetty AK, Upadhya D. Patient-derived iPSC modeling of rare neurodevelopmental disorders: Molecular pathophysiology and prospective therapies. Neuroscience & Biobehavioral Reviews. 2021 Feb 1;121:201-19.
15. Bar S, Schachter M, Eldar-Geva T, Benvenisty N. Large-Scale Analysis of Loss of Imprinting in Human Pluripotent Stem Cells. Cell Rep. 2017 02;19(5):957-68.
16. Stadtfeld M, Apostolou E, Akutsu H, Fukuda A, Follett P, Natesan S, et al. Aberrant silencing of imprinted genes on chromosome 12qF1 in mouse induced pluripotent stem cells. Nature. 2010 May 13;465(7295):175-81.
17. Takikawa S, Ray C, Wang X, Shamis Y, Wu T-Y, Li X. Genomic imprinting is variably lost during reprogramming of mouse iPS cells. Stem Cell Res. 2013 Sep;11(2):861-73.
18. Abi Habib W, Azzi S, Brioude F, Steunou V, Thibaud N, Das Neves C, et al. Extensive investigation of the IGF2/H19 imprinting control region reveals novel OCT4/SOX2 binding site defects associated with specific methylation patterns in Beckwith-Wiedemann syndrome. Hum Mol Genet. 2014 Nov 1;23(21):5763-73.
19. Demars J, Shmela ME, Rossignol S, Okabe J, Netchine I, Azzi S, et al. Analysis of the IGF2/H19 imprinting control region uncovers new genetic defects, including mutations of OCT-binding sequences, in patients with 11p15 fetal growth disorders. Hum Mol Genet. 2010 Mar 1;19(5):803-14.
20. Barroca V, Lewandowski D, Jaracz-Ros A, Hardouin S-N. Paternal Insulin-like Growth Factor 2 (Igf2) Regulates Stem Cell Activity During Adulthood. EBioMedicine. 2017 Feb;15:150-62.
21. Bendall SC, Stewart MH, Menendez P, George D, Vijayaragavan K, Werbowetski-Ogilvie T, et al. IGF and FGF cooperatively establish the regulatory stem cell niche of pluripotent human cells in vitro. Nature. 2007 Aug 30;448(7157):1015-21.
22. Nishino K, Umezawa A. DNA methylation dynamics in human induced pluripotent stem cells. Hum Cell. 2016 Jul;29(3):97-100.
23. Nishino K, Toyoda M, Yamazaki-Inoue M, Fukawatase Y, Chikazawa E, Sakaguchi H, et al. DNA methylation dynamics in human induced pluripotent stem cells over time. PLoS Genet. 2011 May;7(5):e1002085.
24. Tesarova L, Simara P, Stejskal S, Koutna I. The Aberrant DNA Methylation Profile of Human Induced Pluripotent Stem Cells Is Connected to the Reprogramming Process and Is Normalized During In Vitro Culture. PLoS One. 2016;11(6):e0157974.
25. Rulands S, Lee HJ, Clark SJ, Angermueller C, Smallwood SA, Krueger F, et al. Genome-Scale Oscillations in DNA Methylation during Exit from Pluripotency. Cell Syst. 2018 Jul 25;7(1):63-76.e12.
26. Shipony Z, Mukamel Z, Cohen NM, Landan G, Chomsky E, Zeliger SR, et al. Dynamic and static maintenance of epigenetic memory in pluripotent and somatic cells. Nature. 2014 Sep 4;513(7516):115-9.
27. Okuno H, Nakabayashi K, Abe K, Ando T, Sanosaka T, Kohyama J, et al. Changeability of the fully methylated status of the 15q11.2 region in induced pluripotent stem cells derived from a patient with Prader-Willi syndrome. Congenit Anom (Kyoto). 2017 Jul-57(4)-96-103.
28. Nazor KL, Altun G, Lynch C, Tran H, Harness JV, Slavin I, et al. Recurrent variations in DNA methylation in human pluripotent stem cells and their differentiated derivatives. Cell Stem Cell. 2012 May 4;10(5):620-34.
29. Gabory A, Ripoche M-A, Le Digarcher A, Watrin F, Ziyyat A, Forné T, et al. H19 acts as a trans regulator of the imprinted gene network controlling growth in mice. Development. 2009 Oct;136(20):3413-21.
30. Monnier P, Martinet C, Pontis J, Stancheva I, Ait-Si-Ali S, Dandolo L. H19 IncRNA controls gene expression of the Imprinted Gene Network by recruiting MBD1. Proc Natl Acad Sci USA. 2013 Dec 17;110(51):20693-8.
31. Patten MM, Cowley M, Oakey RJ, Feil R. Regulatory links between imprinted genes: evolutionary predictions and consequences. Proc Biol Sci. 2016 Feb 10;283(1824).
32. Stelzer Y, Sagi I, Yanuka O, Eiges R, Benvenisty N. The noncoding RNA IPW regulates the imprinted DLK1-DIO3 locus in an induced pluripotent stem cell model of Prader-Willi syndrome. Nat Genet. 2014 Jun;46(6):551-7.
33. Whipple AJ, Breton-Provencher V, Jacobs HN, Chitta UK, Sur M, Sharp PA. Imprinted Maternally Expressed microRNAs Antagonize Paternally Driven Gene Programs in Neurons. Mol Cell. 2020 02;78(1):85-95.e8.
34. Stadtfeld M, Apostolou E, Ferrari F, Choi J, Walsh RM, Chen T, et al. Ascorbic acid prevents loss of Dlk1-Dio3 imprinting and facilitates generation of all-iPS cell mice from terminally differentiated B cells. Nat Genet. 2012 Mar 4;44(4):398-405, S1-2.
35. Yoshida Y, Takahashi K, Okita K, Ichisaka T, Yamanaka S. Hypoxia enhances the generation of induced pluripotent stem cells. Cell Stem Cell. 2009 Sep 4-5(3)-237-41.
36. Gaignerie A, Lefort N, Rousselle M, Forest-Choquet V, Flippe L, Francois-Campion V, et al. Urine-derived cells provide a readily accessible cell type for feeder-free mRNA reprogramming. Sci Rep. 2018 25;8(1):14363.
37. Yamashita A, Morioka M, Yahara Y, Okada M, Kobayashi T, Kuriyama S, et al. Generation of scaffoldless hyaline cartilaginous tissue from human iPSCs. Stem Cell Reports. 2015 Mar 10;4(3):404-18.
38. Jeziorowska D, Fontaine V, Jouve C, Villard E, Dussaud S, Akbar D, et al. Differential Sarcomere and Electrophysiological Maturation of Human iPSC-Derived Cardiac Myocytes in Monolayer vs. Aggregation-Based Differentiation Protocols. Int J Mol Sci [Internet]. 2017 Jun 1 [cited 2021 Jan 11]; 18(6). Available from: https://www.ncbi.nlm.nih.gov/pmc/articles/PMC5485997/
39. Fontaine V, Duboscq-Bidot L, Jouve C, Hamlin M, Curjol A, Briand V, et al. Generation of iPSC line from MYH7 R403L mutation carrier with severe hypertrophic cardiomyopathy and isogenic CRISPR/Cas9 corrected control. Stem Cell Res. 2021 Apr;52:102245.
40. Gaston V, Le Bouc Y, Soupre V, Burglen L, Donadieu J, Oro H, et al. Analysis of the methylation status of the KCNQ1OT and H19 genes in leukocyte DNA for the diagnosis and prognosis of Beckwith-Wiedemann syndrome. Eur J Hum Genet. 2001 Jun;9(6):409-18.
41. Miller SA, Dykes DD, Polesky HF. A simple salting out procedure for extracting DNA from human nucleated cells. Nucleic Acids Res. 1988 Feb 11;16(3):1215.
42. Azzi S, Steunou V, Rousseau A, Rossignol S, Thibaud N, Danton F, et al. Allele-specific methylated multiplex real-time quantitative PCR (ASMM RTQ-PCR), a powerful method for diagnosing loss of imprinting of the 11p15 region in Russell Silver and Beckwith Wiedemann syndromes. Hum Mutat. 2011 Feb;32(2):249-58.
43. Majid Zamani et al. Humanized Culture Medium for Clinical-Grade Generation of Erythroid Cells from Umbilical Cord Blood CD34+ Cells Adv Pharm Bull, 2021, 11(2), 335-342, doi: 10.34172/apb.2021.031
44. Ludwig, T., Bergendahl, V., Levenstein, M. et al. Feeder-independent culture of human embryonic stem cells. Nat Methods 3, 637-646 (2006). https://doi.org/10.1038/nmeth902
45. Murray E. J. (ed.), Methods in Molecular Biology, Vol. 7, Gene Transfer and Expression Protocols, Humana Press (1991)
46. Johnston SA. Biolistic transformation: microbes to mice. Nature. 1990 Aug 23;346(6286):776-7. doi: 10.1038/346776a0. PMID: 2201923.
47. Brash DE, Reddel RR, Quanrud M, Yang K, Farrell MP, Harris CC. Strontium phosphate transfection of human cells in primary culture: stable expression of the simian virus 40 large-T-antigen gene in primary human bronchial epithelial cells. Mol Cell Biol. 1987 May;7(5):2031-4. doi: 10.1128/mcb.7.5.2031-2034.1987. PMID: 3037341; PMCID: PMC365315.

## Claims

1. A method for obtaining Induced pluripotent stem cells (iPSC) comprising the following steps:
**a.** culture of peripheral blood mononuclear cells (PBMCs) in a first culture medium M1
**b.** transduction or transformation of said PBMCs with a vector comprising at least one Yamanaka factor;
**c.** culture of the transduced or transformed cells obtained in step b) in hypoxic conditions,
**d.** seeding the cultured transduced or transformed cells obtained in step c) onto a matrix gel,
**e.** culture of seeded transduced or transformed cells of step d) in hypoxic conditions into a second culture medium M2,
**f.** replacing the culture medium M2 of the culture step e) by a third culture medium M3 comprising ascorbic acid,
**g.** culturing the transduced or transformed cells obtained in step f) in hypoxic condition thus forming iPSC.

2. The method according to claim 1, wherein the medium M1 comprises at least one compound selected from the group comprising SCF, FLT3, IL3, IL6 and EPO.

3. The method according to any one of claims 1 pr 2, wherein the Yamanaka factor is selected from the group comprising OCT4, SOX2, KLF4 and c-Myc.

4. The method according to any one of the preceding claims, wherein the culture in step c) is carried out in hypoxic conditions wherein the oxygen (O₂) level is from 3% to 17%, preferably from 3 to 7%, more preferably at 5%.

5. The method according to any one of the preceding claims, wherein the culture in step c) is carried out at a temperature from 36°C to 38°C, preferably at 37°C.

6. The method according to any one of the preceding claims, wherein seeding at step d. is carried out at a concentration from 450000 to 500000 cells/ml

7. The method according to any one of the preceding claims, wherein the culture in step e is carried out in hypoxic conditions wherein the oxygen (O₂) level is from 3% to 17%, preferably from 3 to 7%, more preferably at 5%.

8. The method according to any one of the preceding claims, wherein the culture in step e is carried out at a temperature from 36 to 38°C, preferably at 37°C.

9. The method according to any one of the preceding claims, wherein ascorbic acid in medium M3 is at a concentration from 20 to 100 µg/ml, preferably from 30 to 70 µg/ml, more preferably 50µg/ml.

10. The method according to any one of the preceding claims, wherein the process comprises before step c) a seeding step b' of the transduced cells at a concentration from 400000 to 500000 cells/ml.

11. The method according to any one of the preceding claims, wherein step c comprises:
- c1 culturing the transduced cells obtained in step b) for 18 to 28 hours, preferably 24 hours, in hypoxic conditions,
- c2 elimination of the vector from the culture medium,
- c3 resuspending the transduced cells into culture medium M2,
- c4 culturing the resuspended cells of step c3 for 24 to 96 hours, preferably 72 hours.

12. The method according to any one of the preceding claims, wherein the vector for the transduction or transformation of PBMCs is a virus or a plasmid.

13. The method according to any one of the preceding claims, wherein the virus for transduction of PBMCs is Sendai virus.

14. The method according to any one of the preceding claims, wherein the vector for transformation of PBMCs is a plasmid.

15. The method according to any one of the preceding claims wherein the method allows to obtain stable induced pluripotent stem cells (iPSC) with a balanced methylation at imprinting loci.

16. A kit for implementing the method according to any one of claims 1 to 15, comprising a culture medium M1, a matrix gel, a culture medium M2, a culture medium M3 comprising ascorbic acid or an ascorbate or a derivative thereof.

17. The kit as claimed in claim 16, wherein the media M1 to M3 are independently media of clinical grade.
